# EUROPEAN PATENT SPECIFICATION

(11) **EP 1 047 438 B1**
(45) Date of publication and mention of the grant of the patent: **08.09.2004**
(21) Application number: 99900404.7
(22) Date of filing: 08.01.1999
(51) Int. Cl.: A61K 35/78

(54) **A PROCESS FOR THE ISOLATION, RECOVERY AND PURIFICATION OF NON-POLAR EXTRACTIVES**
VERFAHREN FÜR DIE ISOLIERUNG, RÜCKGEWINNUNG UND REINIGUNG VON NICHT-POLAREN EXTRAKTEN
PROCEDE D'ISOLEMENT, DE RECUPERATION ET DE PURIFICATION D'EXTRAITS NON POLAIRES

(30) Priority: 12.01.1998 US 71251 P
(43) Date of publication of application: 02.11.2000
(73) Proprietor: Her Majesty the Queen in Right of Canada as Represented by The Minister of Agriculture and Agri-Food, Ottawa Ontario K1A OC6 (CA)
(72) Inventor: COLLINS, F., William, Ottawa, Ontario K1Y 1J2 (CA); FIELDER, David, A., Ottawa, Ontario K1S 1K7 (CA); SARR, A., Bachir, Ottawa, Ontario K2H 7B4 (CA); REDMOND, Mark, J., Edmonton, Alberta T6G 1B3 (CA); D'ATTILIO, Robert, Z., Ottawa, Ontario K1V 9J9 (CA)
(74) Representative: Harding, Charles Thomas
(86) International application number: PCT/CA1999/000003
(87) International publication number: WO 1999/034810

(56) References cited:
- DE-A- 2 203 884
- DE-A- 2 549 688
- DE-A- 3 045 910
- PATENT ABSTRACTS OF JAPAN vol. 010, no. 194 (C-358), 8 July 1986 & JP 61 036225 A (AIRIN:KK), 20 February 1986
- PATENT ABSTRACTS OF JAPAN vol. 011, no. 184 (C-427), 12 June 1987 & JP 62 005917 A (AIRIN:KK), 12 January 1987

## Description

The present invention relates to the isolation, recovery and purification of non-polar extractives. More specifically the present invention relates to the use of an aliphatic-substituted polysaccharide gel matrix in a process of hydrophobic interaction chromatography, for the absorption and desorption of the extractives in the presence of and as a result of the concentration and selection of an organic solvent.

### BACKGROUND OF THE INVENTION

Agricultural plants, and waste streams from their processing, by way of an example, may contain components that are now being discovered as having desirable therapeutic and other benefits. For example: some saponins have been shown to exhibit antineoplastic chemotherapeutic value (US Patent 5,558,866), while others find use in the treatment of hypercholesterolemia (US Patent 5,502,038). Still further antifungal (e.g. Crombie, W. M. L., and Crombie, L., Phytochemistry 25: 2069-2073, 1986) and immunogenic (US Patent 5,597,807) activities are known as well as surfactant, emulsifying and foam stabilizing properties which are summarized by Price et al. (CRC Crit. Rev. Food Sci. Nutr., 26, 27-135, 1987). These are but a few examples from the literature.

In addition, some flavones and their glycosides are known to exhibit antimutagenicity (e.g. Peryt, B., et al., Mutation Res. 269: 201-215, 1992), and antitumor activity (e.g. Wei, H., et al., Cancer Res. 50: 499-502, 1990). Further reports of beneficial biological activities and functional properties can be found in a number of reviews (e.g."Plant flavonoids in biology and medicine II. Biochemical, cellular, and medicinal properties" Ed. By V. Cody, E. Middleton Jr., J. B. Harborne, and A. Beretz, Liss Inc, New York, 1988.)

Canadian Patent Applications 2,006,957 and 2,013,190 describe ion-exchange processes carried out in aqueous ethanol to recover small quantities of high value byproducts from cereal grain processing waste. According to CA 2,013,190, an alcoholic extract from a cereal grain is processed through either an anionic and/or cationic ion-exchange column to obtain minor but economically valuable products. The anionic, cationic and neutral fractions were analysed by thin-layer chromatography and a number of components were identified. For example in an anionic fraction from an alcoholic extraction of hull-less whole oats, the following components were identified: phenolic acids, including ferulic acid, p-coumaric acid and caffeic acid; alkaloids such as avenanthramides; fatty acids, organic acids and amino acids. From the same alcoholic extract the neutral fraction contained compounds, such as: free sugars; phenolics, such as flavonoids; saponins such as avenacosides, and desglucosyl-avenacosides; alkaloids such as the avenacins; and various polar lipids. The compounds identified in the various fractions were not individually isolated by ion-exchange chromatography since many carried the same net charge under the conditions used and thus, this method alone is of little value in the isolation of these useful components for industrial or commercial use. Furthermore, the extractives to be isolated in the present invention are for the most part neutral under conditions used, and thus cannot be isolated by ion exchange chromatography alone, which sorts molecules according to charge.

PCT application WO 92/06710, discloses both the composition and isolation/separation technologies of Quillaja saponins for end uses as immunogenic complexes, using repeated semi-preparative HPLC on a reverse-phase column with an acetonitrile:water gradient elution. The scale of the separation appears not to be intended for production of significant quantities for commercialization but rather for proof of efficacy. The isolated products were produced only on the microgram scale. The scale-up of the separation technique for commercial applications was not disclosed.

US Patent No. 5,094,960 describes methods of removal of process chemicals from labile biological mixtures by hydrophobic interaction chromatography (HIC) using a resin comprising octadecyl chains coupled to a silica matrix. A method of removing lipid soluble process chemicals such as synthetic detergents, chemical inducers and organic solvents from aqueous biological fluids, particularly directed to producing a protein-containing composition such as blood plasma, cryoprecipitates, and blood plasma fractions, was described. In this disclosure materials and conditions are employed that minimize adsorption and separation of proteins and maximize the removal of the process chemicals. Substantially no biological material is retained on the column. Furthermore, no indication is given as to the intended field of use of any of the compounds and chemicals adsorbed in the process, nor specific conditions to selectively recover any of the adsorbed components retained on the column.

A number of different procedures are known for the isolation and purification of isoflavones. E.D. Walter described a procedure for the preparation of the isoflavone genistin and its aglycone genistein from defatted soybean flakes (J. Amer. Chem. Soc. 63, 3273-3276, 1941). The procedure involved methanol extraction, acetone precipitation, centrifugations and several recrystallizations and gave only one isoflavone, genistin, from which the aglycone genistein could prepared by acid hydrolysis. Ohta et al. described a procedure for isoflavone extraction from defatted soybeans wherein the flakes were extracted with ethanol and the ethanolic extract treated with acetone and ethyl acetate. Column chromatography of the ethyl acetate fraction on silica gel and Sephadex LH-20™ in several additional solvents produced a number of fractions from which individual isoflavones could be recovered by repeated recrystallization (Agric. Biol. Chem. 43: 1415-1419, 1979). Essentially the same separation protocols were used by Farmakalidis, E. and Murphy, P.A. to separate isoflavones extracted using acidified acetone rather than ethanol (J. Agric. Food Chem. 33: 385-389, 1985). These publications are but a few of the many examples in the literature for the laboratory scale extraction and purifications of specific isoflavones. However due to issues of solvent handling and disposal as well as economic feasibility, these procedures are hard to scale up to a commercial process and produce single compounds in undisclosed yields.

US Patent 5,679,806 addressed some of these issues, disclosing a process for the isolation and purification of isoflavones from plant material. The process consisted of three steps whereby the plant material is extracted, the resulting extract fractionated on a reverse phase low pressure polymethacrylate or C₁₈-substituted silica based chromatography column by gradient elution of the adsorbed isoflavones, and finally the resulting fractions containing specific isoflavones were eluted from the column. This process differs in several significant ways from the process described in the embodiments disclosed herein. First, the present process is not restricted to the isoflavone components but also yields a saponin fraction substantially free of isoflavones as well as the entire group of isoflavones which, if desired, can be further fractionated for individual components. Secondly, the present process does not rely on methacrylate or C₁₈-substituted reverse phase inorganic support matrices, which generally display much lower loading capacities and are harder to clean in place than polysaccharide-based gels. Thirdly, the flexibility of the present process allows that conditions be varied, either to capture the isoflavones by absorption or to allow them to elute through the column leaving other non- isoflavone components still absorbed, simply by varying the amount of water in an aqueous alcohol washing solution.

US Patent 5,482,914 teaches that agarose-based gels can be synthesized/modified for the binding of lipoproteins by covalently linking glycidyl ethers of polyoxyethylene detergents of the type HO-(CH₂CH₂O)ₙ-O-R to give a modified gel matrix suitable for the removal of lipoproteins from human and animal body fluids. This technology refers only to the chemical processes for producing the gel and makes no claims either for electrostatic binding of ligands such as we describe, or for any examples of separation or recovery from plant material.

Thus, there is still a need for processes, chromatographic procedures and improved absorption media that are adaptable to a wide range of compounds in a commercially viable manner that provide high concentrations of these compounds which can be subsequently recovered in high yield, purity and in unaltered form. There is also a need for a process in which the chromatographic media can be regenerated and re-used many times to reduce both replacement costs and waste disposal. Furthermore, for commercial scale production of non-polar extractives it would also be advantageous to reduce the direct contact of solvents such as chlorinated hydrocarbons (e.g. chloroform, dichloromethane), nitriles (e.g. acetonitrile), aromatics (e.g. benzene, toluene), other potentially undesirable reagents (e.g. salts, mineral acids, bases), and chromatographic media contaminants (methacrylate-, divinylbenzene-, styrene-monomers, silica etc.) from direct contact with desired products. It is to these ends that this technology is directed.

### SUMMARY OF THE INVENTION

The present invention relates to a process for the isolation, recovery and purification of non-polar extractives or amphiphiles, prepared using one or more extracting solvents, using an aliphatic-substituted polysaccharide gel matrix. More specifically the gel matrix is a covalently-linked, alkyl-substituted neutral polysaccharide gel matrix. The present invention relates to the use of these gels for the absorption and desorption of the extractives in the presence of and as a result of the concentration and selection of an organic solvent in a process of hydrophobic interaction chromatography.

Thus, according to the present invention there is provided a method of isolating a non-polar extractive comprising: contacting said non-polar extractive in an aqueous organic solvent solution with an aliphatic-substituted polysaccharide gel matrix; washing said gel matrix with a first aqueous organic solvent solution; optionally recovering a first extractive from a first effluent stream; washing said gel matrix with a second aqueous organic solvent solution, wherein the proportion of the organic solvent in said second solution is increased over the proportion of organic solvent in said first aqueous organic solvent; and recovering said second extractive from a second effluent stream, wherein the gel matrix consists of a neutral polysaccharide gel matrix of the polyanhydrogalactan class, a neutral polysaccharide gel matrix of the polydextran class or a neutral polysaccharide gel matrix of the hydroxpropyl polydextran class.

In a further embodiment the gel matrix contains covalently linked alkyl substitution of from 4 carbon (ie butyl) to 8 carbon (ie octyl) functions at a 4% or more substitution rate, stable in neutral and mildly acidic or alkaline solutions of said aqueous organic solvent solution , with a molecular size exclusion cut-off range equal to or greater than 10,000 Daltons.

### BRIEF DESCRIPTION OF THE DRAWINGS

These and other features of the invention will become more apparent from the following description in which reference is made to the appended drawings wherein:
**FIGURE 1a** shows the group separation and purification scheme of isoflavones and saponins, and the purification of individual saponins from soy hulls. The HPLC analysis of the isoflavone fraction is shown in **FIGURE 1b.**
**FIGURE 2** is the purification process of saponins from soy flour.
**FIGURE 3** shows the scheme for the isolation and purification of digitonin added to the wheat sample, as per Example 3.
**FIGURE 4** shows the scheme for the isolation and purification of nystatin added to the wheat sample, as per Example 4.
**FIGURE 5** is a typical HPLC tracing of a rice bran steryl ferulate mixture.
**FIGURE 6** shows the structures of Type I steryl ferulates isolated from rice bran.
**FIGURE 7** shows the isolation, recovery and purification of wheat bran flavonoids, alk(en)ylresorcinols and steryl ferulates.
**FIGURE 8** depicts the HPLC profile and structures of wheat bran steryl ferulates.
**FIGURE 9** shows the HPLC profile and example structures of 5-n-alk(en)ylresorcinols from wheat bran.
**FIGURE 10** shows the HPLC profile and structures of major flavonoids of wheat bran.

### DESCRIPTION OF PREFERRED EMBODIMENT

The present invention relates to a process for the isolation, recovery and purification of non-polar extractives, prepared using one or more extracting solvents, using an aliphatic-substituted polysaccharide gel matrix. The present invention relates to the use of these gels for the absorption and desorption of the extractives in the presence of and as a result of the concentration and selection of an organic solvent.

The fundamental principles of both hydrophobic interaction chromatography (HIC) and reverse phase chromatography (RPC) are similar: chromatographic separation of components of a mixture based on their differential affinities between a non-polar or hydrophobic ligand attached to a stationary phase, and a mobile phase. In RPC, the stationary phase usually, but not always, consists of an inorganic, pelliculate or particulate, hydrophilic support, typically silica, onto which a specific ligand with a relatively high degree of hydrophobicity has been introduced at a complete or extremely high substitution rate to effectively replace, mask or resurface the hydrophilic stationary phase. The mobile phase usually, but not always, contains an organic modifier (i.e. organic solvent) typically methanol, acetonitrile, or tetrahydrofuran. In HIC, the stationary phase typically consists of an organic, hydrophilic, macroporous support, typically a chemically modified polysaccharide or polyacrylamide, onto which a specific ligand with a relatively low degree of hydrophobicity, has similarly, but usually to a much lower substitution rate, been introduced to modify but not necessarily eliminate the hydrophilic properties of the support. The mobile phase usually, but not always, consists of water containing a buffer or salt solution of variable concentration.

RPC is now the most commonly used technique for high performance liquid chromatography (HPLC) separation of low molecular weight, relatively stable organic compounds. However, separation of many biological macromolecules (e.g. proteins, peptides and nucleic acids) by RPC has found only limited application, because the stronger interaction with a highly hydrophobic stationary phase and the use of organic solvents as eluent constituents can be very detrimental to the native structure of the macromolecules. As a result of these interactions, most of the macromolecules are subjected to unfolding and denaturation with the concomitant loss of some or all of their biological activity.

HIC has developed as a practical alternative to RPC for the separation and purification of biological macromolecules because both the absorption and desorption precesses can be carried out in an aqueous buffer by simply varying the salt concentrations, conditions that are more favorable to the retention of biological activity of the macromolecule. However, few applications of HIC for the separation of low molecular weight, relatively stable organic compounds appear to have been made. In accordance with the present invention, by maintaining a functional hydrophilic core in addition to the hydrophobic ligand in close spacial proximity, the HIC stationary phase displays unique advantages for the separation and purification of amphiphiles (i.e. compounds containing both hydrophilic and hydrophobic regions) not available in most if not all RPC stationary phase chromatographic media. Examples of such amphiphilic extractives, include but are not limited to saponins, flavonoids and prolamines, which contain hydrophilic substituents (i.e. glycosidic residues) attached to a relatively non-polar backbone. If these amphiphiles additionally show stability towards certain organic solvents such as the lower alcohols and ketones, including ethanol, isopropanol and acetone, these solvents can be used to recover the components bound to the gel (i.e. running the HIC system in an "RPC mode") by either gradient, sequential or batch recovery techniques known by the skilled person in the art of chromatography.

Furthermore, according to the present invention we have observed that such HIC gels possess the capacity to clarify aqueous dispersions and micelles known to be formed when such amphiphiles (i.e. aqueous solutions and dispersions of saponins, flavonoids and prolamines above their critical micellar concentration) are produced artificially or encountered in the extraction, concentration and purification or other manipulation of aqueous alcoholic preparations from agricultural plants and co-processing streams.

The present invention depends on the hydrophobicity of the non-polar extractives to be isolated and the change in hydrophobicity that results from altering the concentration of the recovery solvents, which can be achieved by adding more or less water to the recovery solvent. More specifically, the present invention is based on the observed selective differences in the hydrophobic attraction between relatively non-polar extractives containing aliphatic and/or alicyclic functional groups, as compared to those containing aromatic and/or olefinic functional groups, or neither; and an aliphatically-substituted polysaccharide-based insoluble gel; and on the changes in this attraction that can be made by altering the composition of suitable solvents, simply by the addition of more or less water.

By non-polar extractives, it is meant that the extractives are only partially soluble in aqueous alcoholic solvents ranging in composition from 5% to 95%. This term includes extractives that are relatively non-polar. In one aspect of the present invention the groups of compounds that can be separated are non-polar compounds and are selected from the group, but are not limited to: steroids and triterpenoids, such as saponins, cardiac glycosides and steryl conjugates; flavonoids, such as flavones, flavonols, isoflavones and all of their glycosides; phenolic conjugates, such as aliphatic alcohol, esters and amides; polar lipids, such as mono- and di-glycerides and their derivates and alk(en)ylresorcinols; and prolamines, such as zein, avenin, hordein or gliadin. The non-polar compounds of the present invention include both naturally occurring compounds and synthetic compounds.

By synthetic compounds it is meant any compound prepared by synthetic chemical means. The method of the present invention is particularly useful for the purification of synthetic compounds which have pharmaceutical or therapeutical value.

Naturally occurring compounds include the compounds referred to above, and also include compounds from algae, fungi and unicellular organisms. These naturally occurring compounds include compounds naturally occurring in the microorganism and also those produced by genetically altered cells.

In one embodiment of the present invention, the process is used to isolate soyasaponins. Soyasaponins are sapogenols (triterpenoid aglycones) containing up to five sugars and in some cases have a terminal moiety, tri or tetra acetylated. Saponins in general and soyasaponins in particular are gaining much attention because of the growing market share of soybeans. Soyasaponins have been reported to exhibit haemolytic, goitrogenic, antioxidative and hypolidemic properties. They have also been shown to impart a bitter and astringent taste to soy-based foods. Their isolation and characterisation is important for the breeding of new varieties and for their production as pharmacologically active value-added products. However, because of their complex chemistry, known chemical isolation techniques (i.e., saponification or solvent extraction) may yield low quantities or hydrolysed products. Other examples of useful saponins include the saponins from ginseng and quinoa. Quinoa saponins have demonstrated to be immune system stimulants (US Patents 5,597,807 and 5,688,772).

In a further embodiment of the invention, the process is used to isolate steryl ferulates. Steryl ferulates are naturally occurring ferulic acid esters of plant sterols (i.e. modified triterpene alcohols). They have been identified in the bran oil fraction of a number of monocot cereal grains including wheat, rye, triticale, corn, and rice (e.g. Norton, R. A., Cereal Chem.71: 111-117 1994, and references cited therein). In the oil derived from rice bran, the mixture of steryl ferulates is known as oryzanol or γ-oryzanol and constitutes from about 1.5 to 2.9 % of the total oil composition. The ability of this mixture to reduce serum cholesterol has been reported (Lees, A. M., et al., Atherosclerosis 28: 325-338, 1977; Rong, N., et al., Lipids 32: 303-309, 1997). Processes for its extraction from rice bran oil have been patented (e.g. Takeshi, Y., "γ-oryzanol" German patent #1,301,002 Aug. 14, 1969, Chem. Abstr. 71: 128704r, 1969; Hishashi, S., "Highly concentrated separation of oryzanol by two-step alkali treatment" Japanese patent #76,123,811 Oct. 28, 1976, Chem Abstr. 86: 104664m, 1977). Patents for its formulation (e.g. US Patent #4,612,187) and use in reducing serum cholesterol (US Patent #5,514,398; PCT/EP/96/02344) and in sunscreen formulations (e.g. US Patent #5,817,299) are also known. In the production of oryzanol, solvent extraction, physical refinement and alkali treatment stages lead to mixtures of varying composition due to the crudeness of the process and the complexity of the naturally occurring mixture. From pharmaceutical and therapeutic standpoints, a more definitive isolation, purification and validation system is clearly needed before the biochemical basis of these effects can be better understood.

In one embodiment of the present invention, the compounds can be isolated from plant material. The term plant material includes products of agriculture, viniculture, horticulture or aquaculture. Agricultural plants include cereal grains, for example wheat, oats, rye, corn, rice, quinoa, amaranth, buckwheat, triticale or barley; or oilseeds, such as soybean, canola, flaxseed, sunflower, safflower or mustard; or pulse-crops, for example, peas, lentils or beans; or forage crops, such as fescue, timothy, clover, alfalfa or wheatgrass; or herbs, such as parsley, rosemary, sage, or mint. The compounds can also be recovered from their co-processing streams. The method of the present invention can also be used for the clarification and stabilization of alcohol-water dispersions of cereal and oilseed lipid/protein hazes. However, the invention is not limited to compounds isolated from plants or agricultural co-processing products. The invention can also be used to extract compounds from algae, fungi and unicellular organisms.

The washing solvents, extracting solvents, or recovery solvents, in the context of the present invention these terms are interchangeable, can include but are not limited to the lower alcohols such as methanol, ethanol, propanol or isopropanol; ketones, such as acetone; water, and a combination of the lower alcohol, or ketone, with water.

A person skilled in the art of extraction of naturally-occurring plant constituents will recognize that a number of different extractions methods exist in the literature, including percolation, vat extraction, counter-current extraction, etc. The particular method of extraction used is not important to the process of the present invention.

The present invention uses hydrophobic interaction chromatography alone or in combination with other separation techniques to isolate the compound of interest. In this respect, the invention defined in the present application can be combined with the separation techniques defined in Applicant's co-pending application entitled "The Preparation of Novel Gels for the Purification of Non-Polar Extractives", which uses electrostatically-linked, aliphatic- or alicyclic-substituted anionic or cationic polysaccharide gels prepared from macroporous ionic polysaccharide chromatographic media.

In one embodiment of the present invention the aliphatic-substituted polysaccharide gel matrix typically consist of a neutral polysaccharide of the polyanhydrogalactan class such as cross-linked agarose containing covalently linked alkyl substitution of from 4 carbon (ie butyl) to 8 (ie octyl) carbon functions preferably at 4% or more substitution rate, stable in neutral and mildly alkaline solution of the aqueous organic solvent with a molecular size exclusion cut-off range equal to or greater than 10,000 Daltons.

For example suitable gels include Octyl-Sepharose CL-4B™ and Butyl-Sepharose ™ (Amersham Pharmacia Biotech, Piscataway, NJ).

The neutral polysaccharide gel matrix of the present invention can also include a gel of the hydroxpropyl polydextran class.

The process of purifying the non-polar extractive, according to the present invention involves three basic steps: absorption, washing and recovery. According to one aspect of the present invention, the process involves a fourth optional step of regenerating the column, without the use of a harsh chemical treatment or the generation of excessive salt or non-recoverable processing waste stream.

According to the present invention, separation and purification of a wide range of extractives with similar solubilities in aqueous alcoholic solvents can be effected based on their differential binding to specifically-modified polysaccharide gels. Such extractives can include: steroids and triterpenoids, such as saponins, cardiac glycosides and steryl conjugates; flavonoids, such as flavones, flavonols, isoflavones and all of their glycosides; phenolic conjugates, such as aliphatic alcohol, esters and amides; polar lipids, such as mono- and di-glycerides and their derivates and alk(en)ylresorcinols; and prolamines, such as zein, avenin, hordein or gliadin.

In the case of soybean processing waste, the non-polar extractives consisted of, amongst others, both saponin and isoflavone derivatives which were freely solubilized under the extracting conditions and solvent used. The mixture of saponins present includes at least 4 components consisting of at least one aglycone (no sugars) and 4 glycoside derivatives, as revealed by thin-layer and high performance liquid chromatography, and the mixture of isoflavones consists of at least 12 components consisting of at least 3 different aglycones (no sugars) and at least 9 glycoside derivatives of these aglycones as revealed by thin-layer and high performance liquid chromatography, diode array UV spectroscopy, and mass spectrometry. Even though both classes of natural extractives display similar solubilities in the solvents used, and contain glycosylated and non-glycosylated derivatives, and even though the glycosidic component of both classes in some cases consists of the same sugars; the two classes of natural extractives are clearly separated on the chromatographic gel based on intrinsic differences in their relative attraction or binding to the gel under the conditions used. From similar examples derived from the practice of the invention, it has been established that, due to the preferential retention of compounds containing aliphatic/alicyclic functional groups (i.e. saponins) over those containing aromatic/olefinic functional groups (i.e. flavonoids) at comparable degrees of glycosylation, a separation of the two groups is possible. In practice, the relative preferences and working ranges using an octyl-substituted gel matrix and aqueous ethanol solvent systems have been found, but are not limited to be, as summarized below in Table 1.

**TABLE 1**

| **Relative selectivity and working ranges for separation of saponin and flavonoid derivatives on Octyl Sepharose CL-4B™** | | | |
|---|---|---|---|
| **Relative Non-Polarity (Hydrophobicity)** | **Type and Relative Retention** | | **Range of Solvent EtOH:H**_{**2**}**O (Vol:Vol)** |
| | **Retained** | **Not Retained** | |
| + | | Saponin tetra-, penta-, and hexaglycosides | 0:100 to 10:90 |
| + + | Saponin triglycosides | | 10:90 to 25:75 |
| + + + | Saponin monoglycosides and diglycosides > | Isoflavone and flavonol monoglycosides and diglycosides | 25:75 to 50:50 |
| + + + + | Saponin aglycones (i.e. sapogenins), > sterols, steryl ferulates, alk(en)ylresorcinols | Isoflavone, flavone, and flavonol aglycones, phenolic acids and their amides | 50:50 to 95:5 |

In Table 1, the workable range values are reported as two suitable solvent proportions: the first, for the sorption and washing steps, and the second is the upper range needed for the recovery step. As can be seen in the table, the workable ranges depend on the degree of glycosylation of components being separated but between groups of comparable degree of glycosylation (aglycones, monglycosides, diglycosides, etc.) those containing aliphatic/alicyclic functions (i.e. saponins, sterols, alk(en)ylresorcinols) are preferentially retained by the Octyl Sepharose CL-4B™ gel over those containing aromatic functionality. The proportion of solvent, for example ethanol in the elution solvent solution used for various working ranges increases as the hydrophobicity increases to ensure all components in the mixtures remain soluble, and because less water is needed to effect binding of the compounds to the gel.

Amongst the glycolipids, based on the relative affinities observed in "reverse phase mode of HIC", the order of increasing binding (i.e. requiring less water in the recovery solvent stage) will be diglycosyl monoglycerides ∼ dimonoglycosyl monoglycerides < monoglycosyl monoglycerides < diglycosyl diglycerides < monoglycosyl diglycerides << monoglycerides << diglycerides << triglycerides.

In establishing whether a compound is considered to be bound to the gel and to have exhibited hydrophobic interaction with the gel, the following criteria must be met:
a) it must be soluble in, or form a micelle or stable emulsion in, the solvent with which it is loaded onto the column, and with which the column is washed; and
b) it must be retained by the gel after washing with at least 1.25xV_{b} of the washing solvent, wherein V_{b} is the packed bed volume of the column; wherein the washing solvent is a lower alcohol in combination with water in a ratio sufficient to retain said compound.
This latter criterion must be met since porous gels of the types described herein show molecular size exclusion capabilities. These effects are not however observed beyond approximately 1.25xV_{b} and therefore are not involved in the processes or practices described.

In the recovery step, the proportion of the organic solvent in the aqueous organic solvent solution is increased to decrease the hydrophobic binding of the extractive to the gel and thus elute the extractive. In some embodiments the extractive could be eluted with the initial washing steam.

In the present invention, reference will be made to the degree of relative hydrophobic interaction of specific compounds or groups and/or classes of compounds by the use of a dimensionless constant, K' defined as the ratio of the number of mL of a particular solvent required to move the compounds through a volume of 1 mL of gravity packed gel. Since this dimensionless constant is independent of column dimensions (i.e. length, diameter, etc.), the conditions described herein can be used for scaled up operations over several magnitudes.

As noted previously, as optional fourth step of the present invention is the regeneration of the column, without the use of any harsh chemicals or the generation of excessive salt or non-recoverable processing waste stream. Since conditions for each aplication have been established wherein the compounds of interest have been totally removed from the gel, the column can be regenerated and re-equilibrated in the starting solvent. Surprisingly, it has been found that a simple washing of the gels with a suitable solvent such as 95 % ethanol or isopropanol is sufficient in most cases to remove any material appearing to be adhering to the gels at the end of a process application. The gels are then re-equilibrated with starting solvent for immediate re-use. In this manner, regeneration and recycling up to at least 4000 times over 10 years have been observed without noticeable loss of effectiveness in the processes described herein. Clean-in-place/sanitation procedures where deemed appropriate can be effected using dilute NaOH as per manufacturers recommendations (Amersham Pharmacia Biotech manuals, technical bulletins, etc.).

The purification of the compound, according to the present invention, can be carried out at any suitable temperature, known to persons skilled in the art. The column separations can be accomplished at temperatures ranging from about 2°C to 60°C. Temperature ranges from about 4°C to 30°C, being more commonly used.

### EXAMPLES

### Example 1: Isolation of Soyasaponins and Isoflavones from Soy Hulls using Hydrophobic Interaction Chromatography on Octyl Sepharose CL-4B™

Soybean hulls (*Glycine max* L., selection lines OT93-26 and OT93-28) were finely ground and 25 g samples added with vigorous stirring to 125 mL (solids/liquid = 1/5) refluxing acidified aqueous 80% ethanol (ethanol:water:glacial acetic acid 80:19:1 v:v:v), and heated for 20 additional minutes with continuous stirring under reflux. After cooling to approximately 4° C, the resuspended mixture was transferred to a volumetrically graduated glass column fitted with a coarse porosity fritted disk, and allowed to warm to room temperature (25° C) and settle by gravity to give a packed bed of known volume (V_{b}). The column was then drained and washed with 2xV_{b} fresh acidified aqueous 80% ethanol, and the extraction repeated twice.

After draining, the solid residues were removed and air-dried to constant weight to determine the percentage of material extracted. The combined extracts were reduced to an oily syrup *in vacuo* by rotary evaporation at 40° C.

Hydrophobic interaction chromatography on Octyl Sepharose CL-4B™ was utilized to achieve both group separation of the soysaponins and fractionation/purification of individual soysaponins.

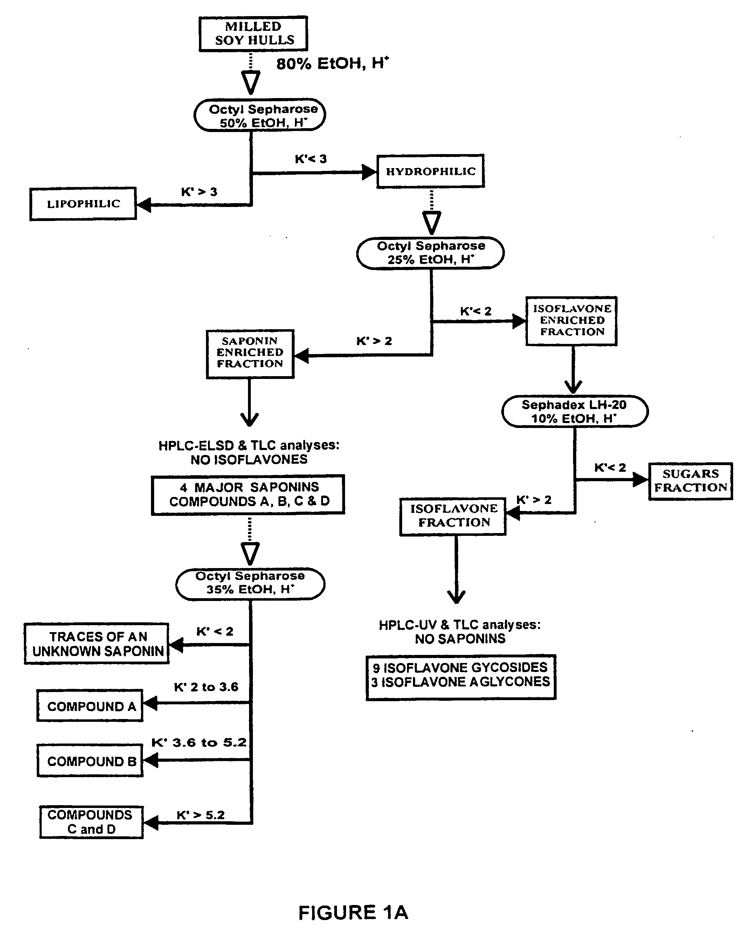

Group separation of the soysaponins from non-saponin, co-extracted glycerides, pigments, flavonoids, amino acids, peptides, and sugars amongst others, was carried out by first, dispersing the oily syrup in 80% ethanol, adding 5 mL of Octyl Sepharose CL-4B™ beads in 80 % ethanol and evaporating the mixture to a thick slurry *in vacuo* by rotary evaporation at 40° C, resuspending the mixture in acidified aqueous 50% ethanol (ethanol:water:glacial acetic acid 50:49.9:0.1 v:v:v), and transferring the slurry to a graduated column of 45 mL V_{b} Octyl Sepharose CL-4B™, pre-equilibrated in the acidified aqueous 50% ethanol solvent. The column (final V_{b} 50 mL) was then washed with 3xV_{b} of the same solvent, to give a washing fraction with K'≤ 3, containing the saponins and isoflavones amongst others, and a K'≥ 3, still hydrophobically bound to the gel. This K'≥ 3 fraction, containing carotenoid pigments, acyl glycerides, free phytosterols, and others, can be recovered and the gel recycled by first passing 2xV_{b} of 95% ethanol to recover the bound material, and then 2xV_{b} of re-equilibrating solvent. The K'≤ 3 fraction was further fractionated on the same column of Octyl Sepharose CL-4B™, pre-eqilibrated in acidified aqueous 10% ethanol (ethanol:water:glacial acetic acid 10:89.9:0.1 v:v:v) by repeating the above procedures of concentrating the K'≤ 3 fraction, adsorbing the fraction to the column and washing the column with 2xV_{b}, except the solvent was acidified aqueous 25% ethanol. The column was pre-equilibrated in acidified aqueous 10% ethanol rather than acidified aqueous 25% ethanol to minimize any chromatographic flow problems arising from density difference between the concentrated sample and the gel. This procedure gave a K'≤ 2 fraction containing all the isoflavones and other components, but devoid of saponins, and a K'≥ 2 fraction hydrophobically bound to the gel containing all the saponins amongst others but devoid of isoflavones. This saponin-enriched K'≥ 2 fraction was recovered simply by increasing the ethanol content of the washing solvent from 25% to 80% (i.e. to decrease the hydrophobic binding of the saponins to the gel) and washing the column with 2xV_{b} of acidified aqueous 80% ethanol. The entire extraction, group separation and purification scheme is shown in Figure 1a. The completeness of this isoflavone/saponin group separation and the identity of the major components of these groups was confirmed by thin layer chromatography (TLC), high performance liquid chromatography (HPLC) and mass spectrometry (MS) analyses of these two fractions using the following protocols.

### Thin-layer chromatography (TLC)

TLC of saponins was performed on MKC₁₈ F reversed phase plates (1x3 in., 200 µm thickness from Whatman International Ltd, Maidstone, England) developed with methanol: aqueous 5% acetic acid (75:25 v:v). Compounds were visualised by spraying with a 0.5% solution (v:v) of *p*-anisaldehyde in acidified aqueous ethanol (ethanol:concentrated sulfuric acid:water:*p*-anisaldehyde 90:5:4.5:0.5 v:v:v:v) and heating at 100° C for 3 min. This reagent gives a number of distinct colors with different constituents including brown (free sugars), transitory yellow quickly turning to pink, green, or grayish-blue (saponins), slowly appearing reddish (amino acids, prolamines), purple (galactosylglycerides), and yellow (lysophosphatides). TLC of isoflavone aglycones and glycosides was carried out on 200µm thick silica gel plastic-backed plates (Baker-Flex 1B2-F, VWR Scientific, Ottawa, Canada) using the following solvent systems: for aglycones, toluene:methyl ethyl ketone:acetic acid (80:15:5 v:v:v); for glycosides, dichloromethane:ethyl acetate:methanol:aqueous 5% acetic acid (40:35:20:5 v:v:v:v). Detection was made using a spray reagent consisting of 0.1 % (w:v) diphenylborinic acid ethanolamine complex (Sigma Chemical Co., St. Louis, MO) in isopropanol and examining the air-dried plate in UV (365nm) light.

### High performance liquid chromatography (HPLC)

HPLC analysis of saponins was conducted using a Thermo Separation Products solvent delivery system and data collection software (PC 1000), a C₁₈ CSC HyperSil column 120Å, 5 µm, (250x4.6mm) operated at a temperature of 25°C, an Alltech Varex MKIII evaporative light scattering detector (ELSD) with the drift tube temperature set at 120°C and the gas flow at 3.06 SLPM. The solvent system consisted of acetonitrile, water and aqueous 5% glacial acetic acid:

| Time | Acetonitrile | H₂O | 5 % acetic acid |
|---|---|---|---|
| 0 | 36 | 57.8 | 6.2 |
| 20 | 36 | 57.8 | 6.2 |
| 25 | 48 | 47 | 5 |
| 30 | 48 | 47 | 5 |
| 35 | 100 | 0 | 0 |
| 40 | 100 | 0 | 0 |
| 45 | 36 | 57.8 | 6.2 |

The flow rate was 1 mL/min.

HPLC analysis of isoflavones was performed using the same column and solvent delivery system but using a Waters 991 photo-diode array UV spectrophotometric detection system (262nm) and accompanying software. The following solvent system was used at a rate of 1mL/min:

| Time | Methanol | 2 % Acetic Acid |
|---|---|---|
| 0 | 30 | 70 |
| 30 | 55 | 45 |
| 40 | 90 | 10 |
| 45 | 100 | 0 |
| 50 | 100 | 0 |
| 55 | 30 | 70 |

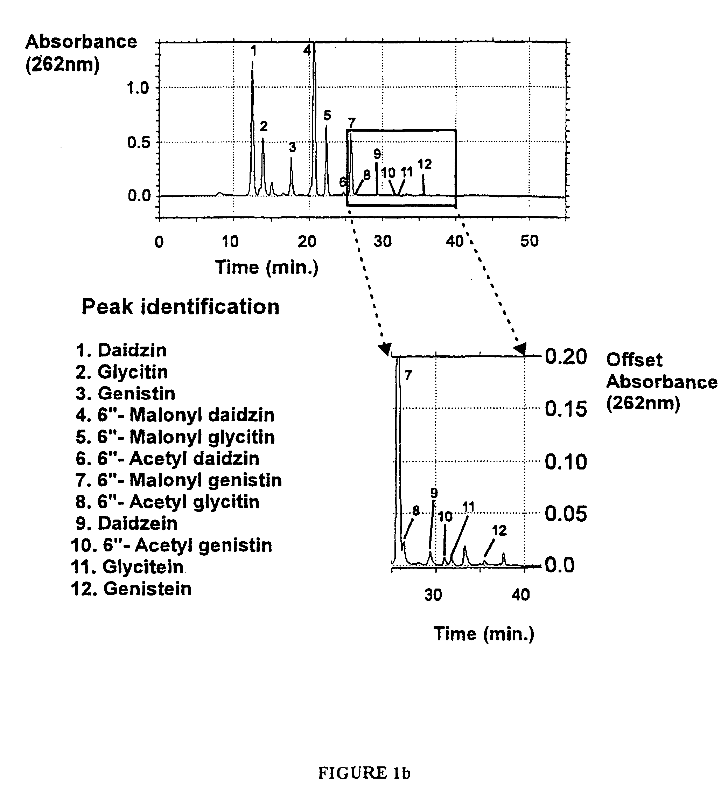

The results are shown in Figure 1b.

### Mass Spectrometry (MS)

Tandem Liquid Chromatography-Mass Spectrometry (HPLC-MS) analyses of pure compounds were performed using flow injection (FIA) with no column. The mobile phase consisted of methanol:water (70:30) and the flow rate was 100 µL/min. Solvents were delivered using a Hewlett Packard 1100 binary pump. Mass spectrometry analyses were conducted using a Micromass Quattro Spectrometer with an upgraded hexapole source operating in both electrospray positive and negative modes. Scanning was done in the range of 200 to 1500 m/z units with a cone voltage of 100 or 220 Volts.

The purification of individual saponins from the saponin-enriched K'≥ 2 fraction was also carried out by hydrophobic interaction chromatography on a 150 mL glass column with a bed volume of 100 mL of Octyl Sepharose CL-4B™ using isocratic elution with acidified aqueous 35% ethanol. Compound A possessed a K' value range from 2 to 3.6 and compound B from 3.6 to 5.2. The entire extraction, group separation and purification scheme is summarized in Figure 1a.

The acidified aqueous ethanol extraction yielded 14% of solubles from soybean hulls. Passage through an Octyl Sepharose CL-4B™ gel with acidified aqueous 50% ethanol separated highly non-polar components such as fats, carotenoid pigments and phosphatides, from the saponins, flavonoids and other more polar components. Further enrichment of the saponin fraction was performed with acidified aqueous 25 % ethanol on the same gel, with highly polar compounds eluting with 2xV_{b} bed volumes leaving the fraction of interest on the column. A third level of purification was performed on QAE Sephadex A-25™ in the acetate form and the fractionation of individual saponins was conducted on Octyl Sepharose CL-4B™ with acidified aqueous 35% ethanol.

Thin layer chromatography analysis of fractions showed 4 distinct spots labelled A, B, C and D. HPLC analysis of fractions showed a peak at 7 min in the fraction with K'<2, peak A and a minor peak not previously detected on TLC and appropriately labelled unknown in K' 2-3, peak A and B in K' 3-4, peak B and remnants of peak A in K' 4-5 and peak C and D in K' >5.

Mass spectrometry positive ion flow injection analysis (FIA) of a purified compound B fraction showed ion peaks at m/z 943, 965 and 987 as [M+H]⁺, [M+Na]⁺ and [M + 2Na]⁺. Negative ion FIA showed a quasi-molecular ion peak at m/z 941. These ions indicated a compound with molecular weight of 942 corresponding to soyasaponin Bb reported by Shiraiwa et al. (Shiraiwa, M., et al., Agric. Biol. Chem. 55, 911-917, 1991) and soyasaponin I by Fuzzati et al. (J. Chromatography A. 777, 233-238, 1997). Soyasaponin I was renamed soyasaponin Bb by Shiraiwa et al. The presence of the major ions reported by Fuzzati et al. with m/z 459, 599, 617, 635, 797 was confirmed. Compound B would then be described as soyasaponin Bb. In addition, compound A was confirmed to be soyasaponin Ba (Shiraiwa et al.) by comparison of TLC and HPLC behaviour with an authentic sample.

The isoflavone-enriched fraction was prepared for HPLC analysis by treatment with Sephadex LH-20™ as follows: The fraction was evaporated *in vacuo* to a thick syrup by rotary evaporation at 40° C, taken up in acidified aqueous 10% ethanol (ethanol:water:glacial acetic acid 10:89.9:0.1 v:v:v), and added to a graduated column of 50 mL Sephadex LH-20™, pre-equilibrated in the acidified 10% aqueous ethanol solvent. The column was then washed with 2xV_{b} of the same solvent, to give a washings fraction with K' ≤ 2, containing the sugars, organic acids and amino acids amongst others, and a K' ≥ 2 fraction , containing the isoflavones, which was recovered from the gel by washing with 2xV_{b} of 80% ethanol. This isoflavone fraction was then used for HPLC analysis. As shown in Figure 1b, the isoflavone fraction contained at least 12 distinct isoflavones of known structure, identified by comparison of TLC, HPLC, and UV spectra with published data (see Wang, H., and Murphy, P. A.,J. Agric. Food Chem. 42: 1666-1673, 1994; Barnes, S., et al. J. Agric. Food Chem. 42: 2466-2474, 1994), along with a number of other peaks all of which showed spectral properties typical of isoflavones.

### Example 2: Isolation of Soyasaponins from Soy Flour using Hydrophobic Interaction Chromatography on Octyl Sepharose CL-4B™

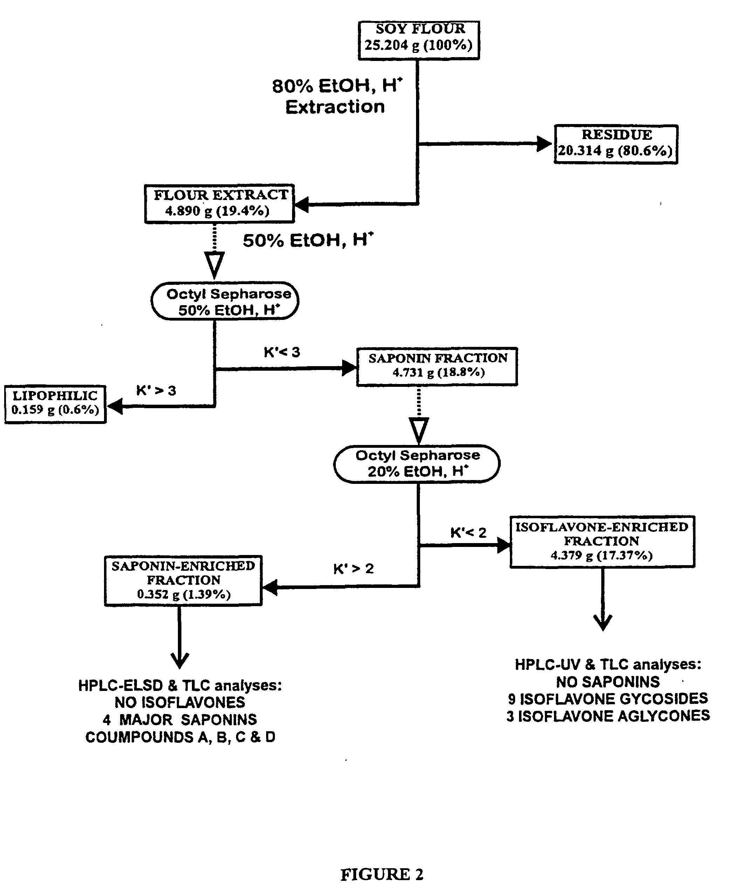

Twenty five g soy flour (Type I, not roasted, defatted, Sigma Chemical Co., St Louis, MO) was added with vigorous stirring to refluxing 80% aqueous ethanol containing 1% glacial acetic acid and extracted, concentrated and fractionated by hydrophobic interaction chromatography on Octyl Sepharose CL-4B™ using essentially the same procedure as described in Example 1 except that the final group separation of soysaponins was carried out in acidified aqueous 20 % ethanol as opposed to 25% ethanol used in Example 1. The isolation scheme is summarized in Figure 2.

Thin layer chromatography and HPLC analyses were performed as in Example 1.

The acidified aqueous ethanol extraction yielded 19.4% (dry basis) of solubles from the soybean flour. As in Example 1, passage through an Octyl Sepharose CL-4B™ gel with acidified aqueous 50% ethanol separated highly non-polar components such as fats, carotenoid pigments and phosphatides (0.63% dry basis), from the saponins, flavonoids and other more polar components to yield a saponin-enriched fraction (18.77% dry basis). Further enrichment of the saponin fraction was performed with acidified aqueous 20% ethanol on the same gel, with highly polar compounds eluting with 2xV_{b} (17.37% dry basis) leaving the fraction of interest on the column. This fraction was then recovered using 80% ethanol containing 0.1 % glacial acidic acid (1.39% dry basis). TLC and HPLC analyses performed on these fractions showed the soysaponin fraction to contain essentially the same components as those revealed in Example 1.

### Example 3: Isolation and Purification of Digitonin from an Artificial Source by Hydrophobic Interaction Chromatography as an Example of the Recovery of a Drug from an Agricultural Crop which has been Genetically Transformed to Produce Pharmaceuticals.

Digitonin is a cardiac glycoside produced by Digitalis purpurea L. (Foxglove, Scrophulariaceae). In modem medicine, digitonin is used to increase the force of the systolic contractions and to prolong the duration of the diastolic phase in congestive heart failure. The generic name is digitonin and the trade name Crystodigin™. It belongs to the drug class of cardiac glycosides (Merck Index #3204, E. Merck, 12th Edition, 1996). The Merck Index describes the content of the commercial product as 70 to 80% digitonin, 10 to 20% gitonin and tigonin, and 5 to 10% minor saponins. The aglycone of digitonin is described as digitogenin [(25R)-5α-Spirostan-2α,3β,15β-triol], and the aglycone of gitonin is described as gitogenin [(25R)-5α-Spirostan-2α,3β-diol]. The aglycone of tigonin is tigogenin [(25R)-5α-Spirostan-3β-ol]. Digitonin and tigonin have 2 glucosyl, 2 galactosyl and one xylosyl residues while gitonin has 2 glucosyl, 1 galactosyl and one xylosyl residues. Thus digitonin and tigonin are pentaglycosides and gitonin is a tetraglycoside.

Although digitonin occurs in Digitalis purpurea L. and is normally extracted and purified from extracts of the aerial parts of this plant, the use of genetically modified plants for the production of this drug represents an alternative that may prove economically more practical (from the standpoints of ease of cultivation, yield, storage, transport, etc.) than current practices. It is not the purpose of the current example to provide an economically practical alternative to these practices, but rather to illustrate the application of the technology described herein for the isolation and purification of the drug from model sources. To this end, the alternative model plant matrix system chosen was a cereal grain (wheat) although in theory, any other suitable matrix would, with minor adaptations, be equally applicable.

### Thin layer chromatography (TLC)

TLC was performed as described in Example 1.

### High performance liquid chromatography (HPLC)

HPLC analyses were conducted using the same equipment as described in Example 1. The solvent system consisted of acetonitrile and H₂O:

| Time | Acetonitrile | H₂O |
|---|---|---|
| 0 | 36 | 64 |
| 10 | 36 | 64 |
| 15 | 48 | 52 |
| 20 | 100 | 0 |
| 30 | 100 | 0 |

The flow rate was 1.0mL/min.

A 25g sample of bread wheat (composite sample Canadian Hard Red Winter Wheat) was finely ground in a coffee mill and 25mg digitonin (Sigma Chemical Co., St. Louis , MO) thoroughly mixed in with the grounds. The mixture was then carefully added with vigorous stirring to 125mL (solids/ liquids = 1/5 w:v) of refluxing aqueous 80% acetone and extracted with stirring for a further 20 minutes. After cooling to room temperature, the mixture was centrifuged (2830xg, 15 min.) and the supernatant decanted. The pellet was then resuspended in 125mL of fresh aqueous 80% acetone, re-extracted twice and the combined supernatants, constituting the extract, evaporated *in vacuo* by rotary evaporation at 40° C to an oily residue.

This extract was taken up in 5mL of acidified aqueous 50% ethanol (ethanol:water:glacial acetic acid 50:49:1 v:v:v) and loaded onto a graduated glass column of Octyl Sepharose CL-4B™ gel, pre-equilibrated and gravity-packed (final packed V_{b} = 100 mL; i.e. 4mL gel/gm wheat extracted) in the same acidified aqueous 50% ethanol solvent. The column was then washed with 3xV_{b} of acidified aqueous 50% ethanol to give a K' ≤ 3 fraction which was concentrated *in vacuo* at 40° C by rotary evaporation to a brownish syrup. TLC analyses showed this fraction contained digitonin, sugars, amino acids, organic acids, some of the pigments and some of the prolamines amongst others. The material remaining on the column was removed (and the column regenerated for re-use), by passing 3xV_{b} of 95% ethanol to give a K' ≥ 3 fraction. TLC of this fraction revealed only traces of the digitonin and contained the bulk of the polar lipids, the lipophilic prolamine proteins, and some of the pigments, amongst others. The completeness of the separation of digitonin between the K' ≤ 3 fraction and the K' ≥ 3 fraction was examined by quantitative HPLC using an authentic sample of digitonin prepared and characterized (TLC, HPLC, MS) from the commercial sample. Greater than 96% of the digitonin was recovered in the K' ≤ 3 fraction with less than 4% in the K' ≥ 3 fraction.

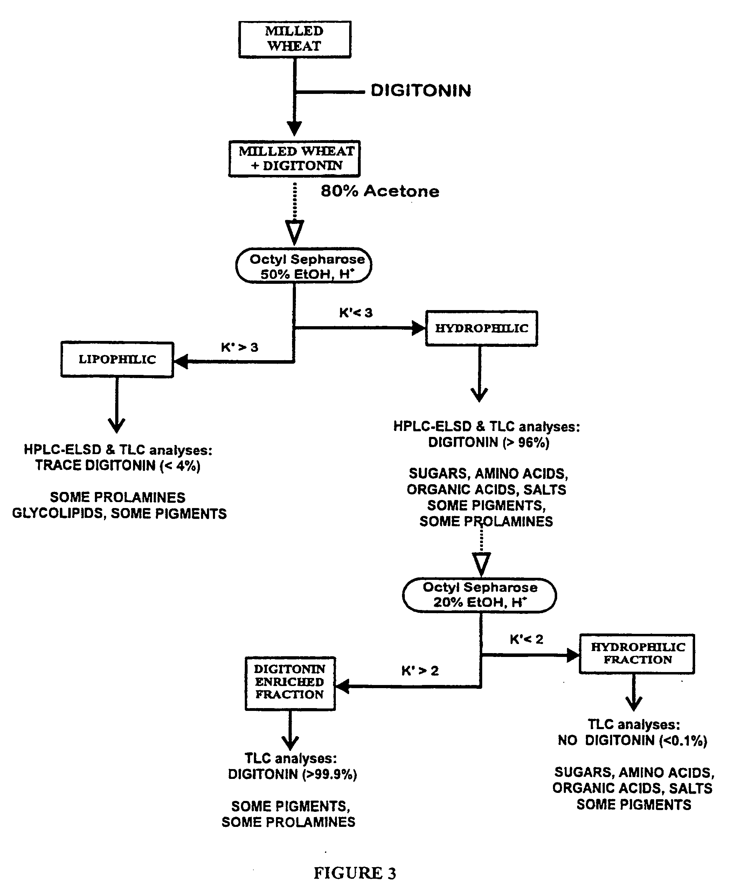

Further purification of the digitonin in the K' ≤ 3 fraction from other components was carried out using HIC simply by re-running the 50% ethanol K' ≤ 3 fraction through the column using a solvent with a higher water content to increase the binding of the cardiac glycoside to the gel. Thus, the K' ≤ 3 fraction was taken up in 5mL of acidified aqueous 20% ethanol (ethanol:water:glacial acetic acid 50:49:1 v:v:v) and loaded onto a graduated glass column of Octyl Sepharose CL-4B™ gel, pre-equilibrated and gravity-packed (final packed V_{b} = 100 mL; i.e. 4mL gel/gm wheat extracted ) in the same acidified aqueous 20% ethanol solvent. The column was then washed with 2xV_{b} of acidified aqueous 20% ethanol to give a K' ≤ 2 fraction containing the sugars, amino acids, and organic acids amongst others, which was concentrated *in vacuo* at 40° C by rotary evaporation to a brownish syrup. The material remaining on the column was removed (and the column regenerated for re-use), by passing 2xV_{b} of 95% ethanol to give a K' ≥ 2 fraction, containing the digitonin, amongst others. The completeness of the separation of digitonin between the K' ≤ 2 fraction and the K' ≥ 2 fraction was examined by quantitative TLC. The digitonin was found in the K'≥ 2 and no detectable digitonin occurred in the K'≤ 2 fraction. The scheme for the isolation and purification of digitonin added to the wheat sample is summarized in Figure 3.

### Example 4: Isolation and Purification of Nystatin from an Artificial Source by Hydrophobic Interaction Chromatography as an Example of the Recovery of a Drug from an Agricultural Crop which has beenGenetically Transformed to Produce Antimicrobials.

Nystatin is an antifungal antibiotic produced by Streptomyces spp. (Merck Index #6834, E. Merck, 12th Edition, 1996). which has established itself in human therapy as a valuable agent for the treatment of both local and internal infections of molds and yeasts such as *Candida albicans*. It belongs to a group of macrocyclic lactone antibiotics containing a conjugated polyene system and a glycosidically-linked aminodesoxyhexose which, in combination with a free carboxylic group give the compound zwitterionic properties. A number of trade names are known including Fungicidin™ and Mycostatin™. Although nystatin occurs in Streptomyces spp. and is normally extracted and purified from extracts of cultures of this bacterium, the use of genetically modified plants for the production of this drug represents an alternative that may prove economically more practical (from the standpoints of ease of cultivation, yield, storage, transport, etc.) than current practices. It is not the purpose of the current example to provide an economically practical alternative to these practices, but rather to illustrate the application of the technology described herein for the isolation and purification of the drug from model sources. To this end, the alternative model plant matrix system chosen was a cereal grain (wheat) although in theory, any other suitable matrix would, with minor adaptations, be equally applicable.

Nystatin was obtained from Sigma Chemical Co., St Louis, MO. Sepharose™ and Sephadex™ based gels were obtained from Pharmacia. All solvents and reagents were of the highest purity commercially available.

### Thin layer chromatography (TLC)

TLC was performed as described in Example 1.

A 25g sample of bread wheat (composite sample Canadian Hard Red Winter Wheat) was finely ground in a coffee mill and 25mg nystatin (Sigma Chemical Co., St. Louis , MO) thoroughly mixed in with the grounds. The mixture was then carefully added with vigorous stirring to 125mL (solids/ liquids = 1/5 w:v) of refluxing acidified aqueous 80% ethanol (ethanol:water:glacial acetic acid 80:19:1 v:v:v) and extracted with stirring for a further 20 minutes. After cooling to room temperature, the mixture was centrifuged (2830xg, 15 min.) and the supernatant decanted. The pellet was then resuspended in 125mL of fresh acidified aqueous 80% ethanol, re-extracted twice and the combined supernatants, constituting the extract, evaporated *in vacuo* by rotary evaporation at 40° C to an oily residue.

This extract was taken up in 5mL of acidified aqueous 50% ethanol (ethanol:water:glacial acetic acid 50:49:1 v:v:v) and loaded onto a graduated glass column of Octyl Sepharose CL-4B™ gel, pre-equilibrated and gravity-packed (final packed V_{b} = 100 mL; i.e. 4mL gel/gm wheat extracted) in the same acidified aqueous 50% ethanol solvent. The column was then washed with 3xV_{b} of the acidified aqueous 50% ethanol to give a K' ≤ 3 fraction which was concentrated *in vacuo* at 40° C by rotary evaporation to a brownish syrup. TLC analyses showed this fraction contained nystatin, sugars, amino acids, organic acids, some of the pigments and some of the prolamines amongst others. The material remaining on the column was removed (and the column regenerated for re-use), by passing 2xV_{b} of aqueous 80% ethanol and 2xV_{b} of aqueous 95 % ethanol. TLC of these fractions revealed no traces of the nystatin and contained the bulk of the polar lipids, the lipophilic prolamine proteins, and some of the pigments.

Further purification of the nystatin in the K' ≤ 3 fraction from other components was carried out using HIC simply by re-running the 50% ethanol K' ≤ 3 fraction through the column using a solvent with a higher water content to increase the binding of the nystatin to the gel. Thus, the K' ≤ 3 fraction was taken up in 5mL of aqueous 1% acetic acid and loaded onto a graduated glass column of Octyl Sepharose CL-4B™ gel, pre-equilibrated and gravity-packed (final packed V_{b} = 100 mL; i.e. 4mL gel/gm wheat extracted ) in the same solvent. The column was then washed with 2xV_{b} of 1 % acetic acid to give a K' ≤ 2 fraction which was concentrated *in vacuo* at 40° C by rotary evaporation to a brownish syrup. The material remaining on the column was removed (and the column regenerated for re-use), by passing 2xV_{b} of 80% ethanol to give a K' ≥ 2 fraction. Qualitative TLC analyses of the K' ≤ 2 fraction showed it contained the sugars, amino acids, organic acids and some of the pigments. Similar analyses of the K' ≥ 2 fraction revealed the presence of nystatin and some of the pigments amongst others. The completeness of the separation of nystatin between the K' ≤ 2 fraction and the K' ≥ 2 fraction was examined by quantitative TLC. All (i.e. > 99.9 %) of the nystatin was found in the K' ≥ 2 and no detectable (i.e. <0.1%) nystatin occurred in the K' ≤ 2 fraction.

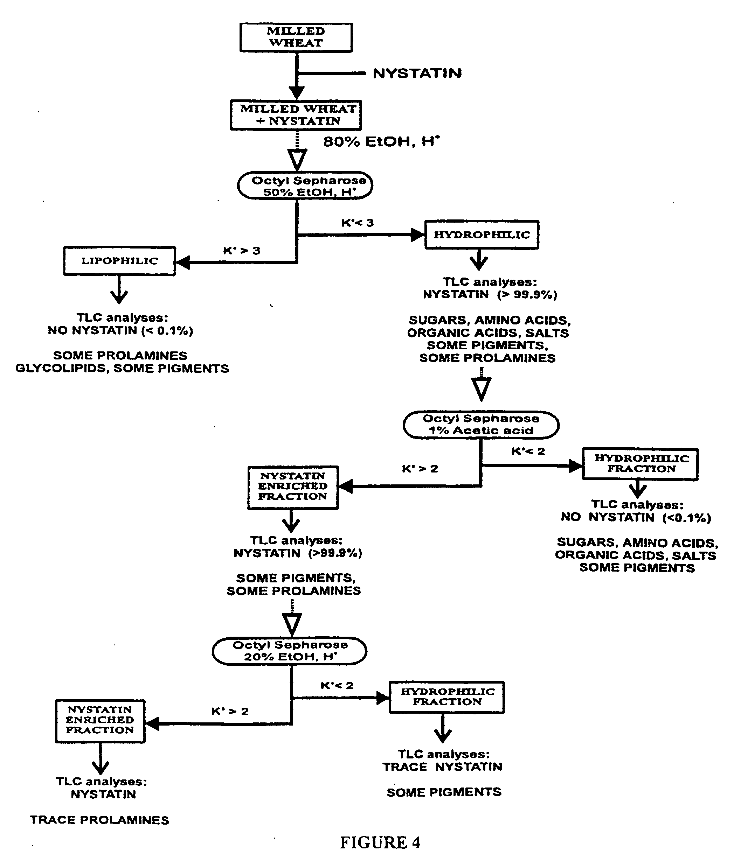

Finally, still further purification of the nystatin was achieved by HIC at an ethanol: water ratio intermediate between the above steps. Thus, the K' ≥ 2 fraction was taken up in 5mL of acidified aqueous 20% ethanol (ethanol:water:glacial acetic acid 20:79:1 v:v:v) and loaded onto a graduated glass column of Octyl Sepharose CL-4B™ gel, pre-equilibrated and gravity-packed (final packed V_{b} = 100 mL; i.e. 4mL gel/gm wheat extracted ) in the same solvent. The column was then washed with 2xV_{b} of acidified aqueous 20% ethanol to give a K' ≤ 2 fraction which was concentrated *in vacuo* at 40° C by rotary evaporation to a brownish syrup. The material remaining on the column was removed (and the column regenerated for re-use), by passing 2xV_{b} of 80% ethanol to give a K' ≥ 2 fraction. Qualitative TLC analyses of the K' ≤ 2 fraction showed it contained the remainder of the pigments but only traces of nystatin. The K' ≥ 2 fraction, on the other hand, contained almost all of the nystatin in a highly purified form. The scheme for the isolation and purification of nystatin added to the wheat sample is summarized in Figure 4.

### Example 5: Isolation and Purification of Steryl Ferulates from Rice Bran using Hydrophobic Interaction Chromatography on Octyl Sepharose CL-4B™

All procedures were carried out in subdued light to prevent the light-induced E-Z isomerization of the steryl ferulates. Rice bran was extracted with 95 % ethanol by gradually adding, with vigorous stirring, 300 g of bran to 1.5 L (solids/liquid = 1/5) of refluxing 95 % ethanol and allowing the mixture to cool with continued stirring to approximately 4°C. The stirred mixture was then transferred to a volumetrically graduated 2 L glass funnel fitted with a coarse fritted disk and a stopcock. The slurry was left to equilibrate to room temperature and settle by gravity to give a packed bed and supernatant liquid extract of known volumes. The supernatant extract was drained and the packed bed washed with a further 2xV_{b} of fresh 95 % ethanol. The combined extract and washings were then concentrated *in vacuo* by rotary evaporation at 40° C to an oily syrup. Further drying by lyophilization gave 27.54 g of yellow-green foam ( yield: 9.18%).

A highly enriched steryl ferulate fraction was then prepared from the crude extract by Hydrophobic Interaction Chromatography on Octyl Sepharose CL-4B™ as follows: To the yellow-green foam, 125 mL Octyl Sepharose CL-4B™ pre-equilibrated in 95 % ethanol was added and the mixture stirred to dissolve the syrupy extract and slurry the gel (adsorbing ratio: ∼ 13 mL gel /g extract or ∼ 0.42 mL gel/ g rice bran extracted). The slurried mixture was then concentrated *in vacuo* to remove as much of the solvent as possible, slurried again in aqueous 50% isopropanol and carefully added to a 500 mL volumetrically graduated column containing a 125 mL Octyl Sepharose CL-4B™ gel bed, pre-equilibrated and gravity-packed in aqueous 50% isopropanol. After settling, the combined absorbent gel layer and prepacked gel bed volume (i.e. V_{b}) was ∼250 mL. The supernatant liquid above the newly constituted 250 mL gel bed was drawn off, the column washed with a further 2xV_{b} of fresh 50% aqueous ethanol, and the combined eluent and washings concentrated *in vacuo* to give a thick, yellow-brown resin by rotary evaporation at 40° C. The steryl ferulate-enriched fraction absorbed on the gel was then recovered with 2xV_{b} of 100% isopropanol.

Further purification of the steryl ferulates from other lipophilic compounds was achieved by double ion exchange chromatography using QAE Sephadex A-25™. Since the steryl ferulates have no net charge at or below pH 6, they will not be retained on an anion exchange matrix whereas lipids of the classes including free fatty acids, acidic phosphatides, amino acids, peptides, and phenolic acids will be retained and can thus be removed from the mixture. Furthermore, since the steryl ferulates have a net negative charge at or above pH 8, they will be retained on an anionic exchange matrix whereas all remaining neutral lipids will pass through the matrix. The retained steryl ferulates can then be recovered from the column by eluting the column with a mildly acidic recovery solution of pH 6 or less.

Accordingly, a volumetrically graduated column of QAE Sephadex A-25™ in the trifluoroacetate form was prepared by first converting 100 mL of the gel as received from the manufacturer in the Cl⁻ form, to the OH⁻ form with 1N NaOH in aqueous 50% ethanol, and then converting the OH⁻ form to the trifluoroacetate form with 1% trifluoroacetic acid in 50% ethanol (ethanol:water:trifluoroacetic acid 50:49:1). The column was then washed with aqueous 50% ethanol until the pH of the washings was ∼6. Finally, the column was equilibrated in the solvent isopropanol:methanol:dichloromethane:water (50:25:22.5:2.5 v:v:v:v), resuspended and allowed to settle to give a bed volume (V_{b}) of ∼75 mL.

The steryl ferulate fraction prepared above was evaporated to dryness *in vacuo* by rotary evaporation at 40°C, and dissolved in 10 mL of isopropanol:methanol:dichloromethane:water (50:25:22.5:2.5 v:v:v:v). The solution was absorbed onto the column and washed with 2xV_{b} of fresh solvent to give a washing fraction highly enriched in the steryl ferulates amongst others. The anionic material adsorbed on the column, including free fatty acids, acidic phosphatides, amino acids, peptides, and phenolic acids was then recovered using 2xV_{b} of the solvent isopropanol:methanol:dichloromethane:5% aqueous trifluoroacetic acid (50:25:22.5:2.5 v:v:v:v), and the column recycled by washing the column and equilibrating to pH∼6 using 2xV_{b} of isopropanol:methanol:dichloromethane:water (50:25:22.5:2.5 v:v:v:). The neutral washing fraction was concentrated to *dryness in vacuo* by rotary evaporation at 40°C to give a yellowish-green oil, dissolved in 10 mL of isopropanol:methanol:dichloromethane:water (50:25:22.5:2.5 v:v:v:v), and absorbed onto a 75 mL V_{b} QAE Sephadex A-25™ column in the OH⁻ form, prepared as described above and equilibrated in isopropanol:methanol:dichloromethane:water (50:25:22.5:2.5 v:v:v:v). The column.was then washed with 2xV_{b} of fresh solvent to give a washing fraction consisting of, amongst others, neutral glycerides, wax alcohols and free and glycosylated sterols and their aliphatic esters. The absorbed material was recovered by passing 2xV_{b} of the solvent isopropanol:methanol:dichloromethane:5% aqueous acetic acid (50:25:22.5:2.5 v:v:v:v), and concentrating the fraction *in vacuo* by rotary evaporation at 40°C to give 1.17 g of a pale green foam containing >98% purity (HPLC, TLC, UV and MS) steryl ferulates (yield: 0.39% of dry bran extracted).

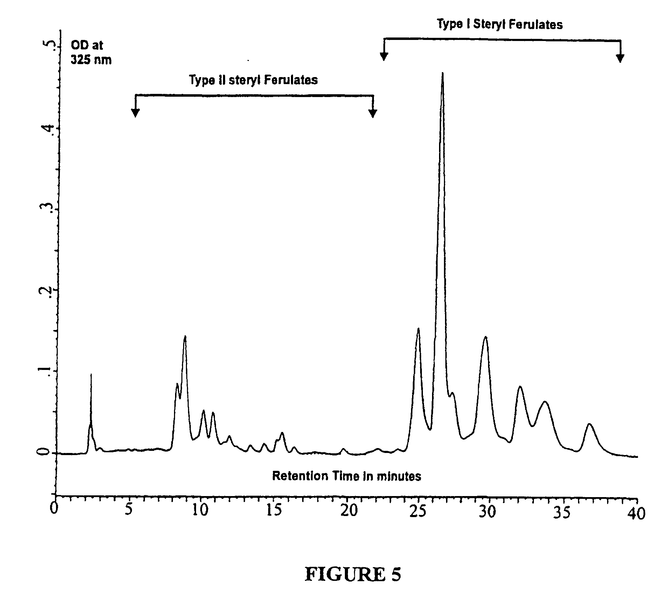

HPLC analysis of the steryl ferulate mixture showed the presence of at least 18 distinct components all of which exhibited the characteristic UV absorption spectrum of either an E- or Z-feruloyl moiety. A typical HPLC tracing of the mixture of steryl ferulates prepared above is shown in Figure 5. To facilitate analyses, the mixture was arbitrarily separated into 2 types, Type I and Type II steryl ferulates, by further Hydrophobic Interaction Chromatography on Octyl Sepharose CL-4B™. The pale green foam prepared above (1.17 g) was dissolved in 15 mL of 95 % ethanol and ∼40 mL of Octyl Sepharose CL-4B™, pre-equilibrated in 95% ethanol, added. After thorough mixing, the slurry was concentrated *in vacuo* by rotary evaporation at 40°C to remove the ethanol, redispersed in 65% acetone, transferred with washings to a column of 85 mL Octyl Sepharose CL-4B™ pre-equilibrated in 65% acetone, and allowed to settle by gravity (final V_{b} 125 mL). The column was then washed with 3.5xV_{b} of fresh 65% acetone and the washings evaporated to dryness *in vacuo* by rotary evaporation at 40° C, to give Type II steryl ferulates (0.018 g pale green foam; yield: 0.006% of dry bran extracted). The Type I steryl ferulates were then recovered from the column with 2xV_{b} isopropanol and evaporated to dryness *in vacuo* by rotary evaporation at 40°C, to give 0.972 g of pale green foam (yield: 0.32% of dry bran extracted).

The structural identification, qualitative and quantitative analyses of the rice bran steryl ferulate mixture were carried out on the Type I steryl ferulates, using the following methods:

### High Performance Liquid Chromatography (HPLC):

Semi-preparative HPLC was carried out on a Beckman 110A pump fitted with a Rheodyne 7125 injector and a 200µL sample loop. The HPLC was run in an isocratic mode with 100% methanol at 2.0 mL/min flow rate through a CSC ODS-2 column (10µm, 9.4x250 mm). Injection volume was 50µL (i.e. 8.4 mg) into the 200 µL sample loop. The eluent was monitored at 325 nm with a Hewlett-Packard 8452 diode array spectrophotometer.

Analytical runs were performed on a Thermo Separation Products P4000 quaternary pump fitted with a Rheodyne 7125 injector and 20 µL sample loop. Separations were effected on a CSC ODS-2 column (5µm, 4.0x250 mm), using a mobile phase of methanol:acetonitrile:isopropanol:water (45:45:5:5 v:v:v:v) which was ramped to methanol:acetonitrile:isopropanol (45:50:5 v:v:v) from 6 to 10 minutes and held constant for 30 minutes. The flow rate was 1.0 mL/min and the temperature was maintained at 20° C with a Varian 2080 column oven.

### Thin-Layer Chromatography:

Thin-layer chromatography was performed on both silica gel plates (Baker-Flex, 1B2-F), in the normal phase mode, with n-butyl acetate:cyclohexane (20:80 v:v) as solvent, and C₁₈ precoated plates (Whatman, MK C₁₈ F, 200 µm) with 100% methanol as solvent. Steryl ferulates were visualized by examination of the plates under UV (365nm) before and after spraying with a 5 % solution of ethanolamine in isopropanol (v:v). The steryl ferulates were visible as dark UV-absorbing spots before spraying, turning sky blue with ethanolamine.

### Spectral Analyses:

UV data was provided by a Waters 991 Photodiode Array Detector. NCI mass spectra were obtained using flow injection on a Hewlett Packard 1090 HPLC coupled to a 5988 HP mass spectrometer via a thermospray interface. EI mass spectra were acquired by direct inlet solid probe analysis on a Finnigan 4500 spectrometer with an ionization potential set to 30 eV. NMR spectra were acquired on a Bruker AM 500 spectrometer operating at 500 MHZ and 125.7 MHZ, respectively, for ¹H and ¹³C. Spectra were recorded in CDCl₃ and referenced to CHCl₃ at 7.24 ppm ( ¹H) and CDCl₃ at 77.0 ppm (¹³C). All chemical shifts are reported relative to tetramethylsilane. ¹H assignments were aided by ¹H/¹³C heteronuclear correlation spectra (HETCORR) and carbon resonance assignments by DEPT and HETCORR NMR experiments. Melting points were recorded on an Electrothermal apparatus and are uncorrected.

### Quantitative Analyses:

Quantization of the steryl ferulates was carried out on a Perkin Elmer LC-85B Spetrophotometric Detector monitoring at 326nm with the attenuation set at 0.08 AU units and the detector response set at 20 ms. Integration was performed on Thermo Separations WOW software. Several standards of 24-methylenecycloartanol-3β-O-E-ferulate were prepared from material recrystallized from semi-preparative HPLC runs (colourless needles from aqueous isopropanol, mp: 193-194.5°C) and used to construct a calibration curve. The response factor was applied to all types of steryl ferulates and consequently any concentrations were reported in terms of 24-methylenecycloartanol-3β-O-E-ferulate equivalents. All standards and samples were stored at 4° C in the dark and were stable for several months (no detectable indication of E/Z isomerization).

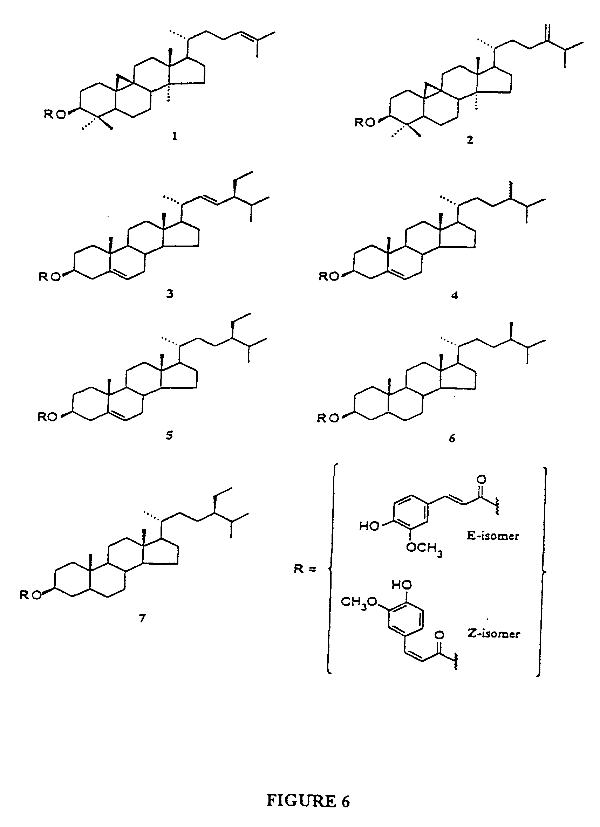

Using repeated injections of the mixture of Type I steryl ferulates, individual components were isolated by semi-preparative HPLC and their purity monitored by analytical HPLC and TLC. When purified, pooled samples were concentrated *in vacuo* by rotary evaporation and crystallized from methanol. Sub-samples of each pure compound were dissolved in methanol:water (95:5 v:v) and exposed to UV light (365 nm) for 30 minutes in a Chromatovue chamber (Ultraviolet Products Ltd., Sacramento, CA) to induce E/Z isomerization and allow the production and characterization of both isomeric forms of each steryl ferulate. The identification, relative retention times (RRₜ) and concentration of Type I steryl ferulates from the rice bran are summarized in Table 2 and their structures are shown in Figure 6.

**TABLE 2**

| **Composition of Type I Steryl Ferulates from Rice Bran** | | | |
|---|---|---|---|
| **Compound** | **Structure** | **RR**_{**t**}***** | **Concentration mg/100 g**** |
| Cycloartenyl-*E*-ferulate | (I*E*) | 0.9434 | 34 |
| Cycloartenyl-*Z*-ferulate | (1*Z*) | 0.9718 | nd |
| 24-methylenecycloartanyl-*E*-ferulate | (2*E*) | 1.000 | 87 |
| 24-methylenecycloartanyl-*Z*-ferulate | (2*Z*) | 1.038 | 14 |
| Stigmasteryl-*Z*-ferulate | (3*Z*) | 1.076 | nd |
| 24ξ-methylcholesteryl-*Z*-ferulate | (4*Z*) | 1.088 | nd |
| Stigmasteryl-*E*-ferulate | (3*E*) | 1.110} | 49 |
| 24ξ-methylcholesteryl-*E*-ferulate | (4*E*) | 1.120} | |
| Sitosteryl-*Z*-ferulate | (5*Z*) | 1.180 | nd |
| Sitosteryl-*E*-ferulate | (5*E*) | 1.213 | 24 |
| Campestanyl-*Z*-ferulate | (6*Z*) | 1.229 | nd |
| Campestanyl-E-ferulate | (6*E*) | 1.272 | 26 |
| Stigmastanyl-*Z*-ferulate | (7*Z*) | 1.341 | 2 |
| Stigmastanyl-*E*-ferulate | (7*E*) | 1.388 | 13 |
| | | | |
| Total Type I steryl ferulates | | | 249 |

| | | | |
|---|---|---|---|
| * Relative Retention Times (RRₜ) relative to 24-methylenecycloartanol-*E*-ferulate (absolute retention time =26.54 min) | | | |
| ** Concentration reported in 24-methylenecycloartanol-*E*-ferulate equivalents; nd = not detected | | | |

Preliminary analyses of the Type II steryl ferulates suggest that they are the more unsaturated analogues and isomers of the Type I ferulates containing one or two additional double bonds (e.g. cyclobranol) as seen by their mass spectra which exhibit comparable fragments at 2 and in some cases 4 mass units less than those exhibited by the Type I ferulates.

The use of Hydrophobic Interaction Chromatography on alkyl substituted polysaccharide gels for the separation of steryl ferulates offers an improved method when used in combination with ion-exchange chromatography for the preparation of these non-polar compounds over available methods. The porous nature of the polysaccharide gel matrix gives it a much higher loading capacity than conventional non-porous silica-based media. Typical loading values of 50-75 mg of crude extracts per mL of gel media represent an increase of from 10 to 50 fold over conventional products and are comparable to loading capacities of synthetic resins such as those based on modified polystyrene and polymethacrylate matrices (Takayanagi, H. et al., "Non-ionic adsorbents in separation processes" In, *Downstream Processing of Natural Products, A Practical Handbook.* Ed. by Michael S. Verall, John Wiley & Sons, Toronto, 1996. pp. 159-178).

### Example 6: Isolation and Purification of Wheat Bran Flavonoids, 5-n-alk(en)ylresorcinols and Steryl Ferulates using Hydrophobic Interaction Chromatography on Octyl Sepharose CL-4B™

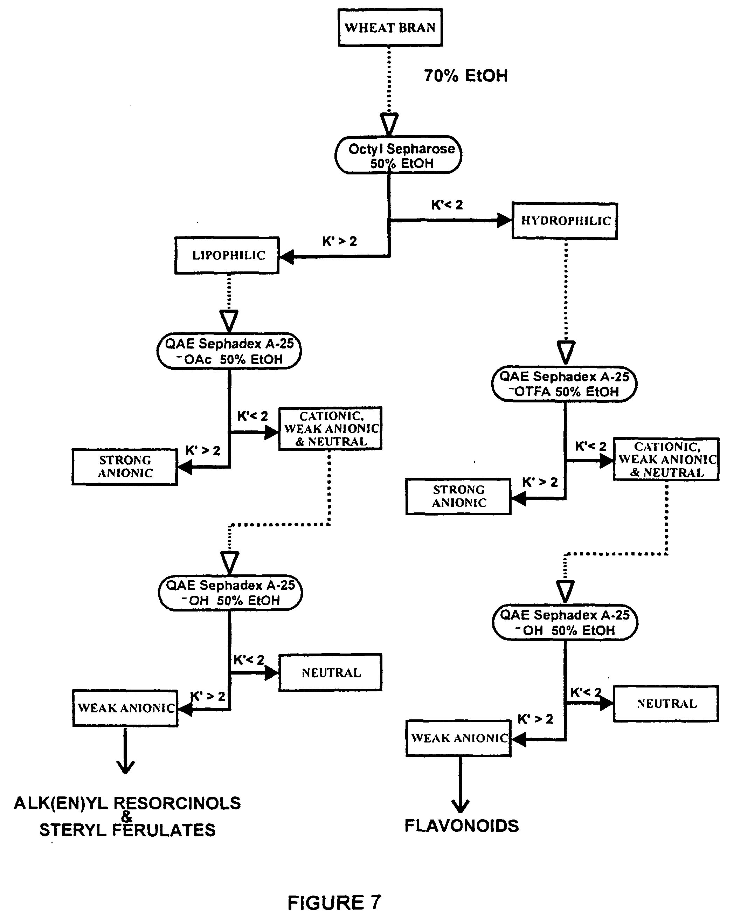

All extractions, fractionations and analyses were performed under subdued room lighting conditions with special attention paid to elimination of UV light from all solution chemistry to prevent the E-Z isomerization of the steryl ferulates. The isolation and purification procedure is summarized in Figure 7. Wheat bran (composite sample, from light white wheat, "Product B", E. Timm & Sons, Goole, U.K.) was ground in a Tekmar A-10 analytical mill for 20 seconds to reduce particle size to ≤ 400 mesh. To 100 mL refluxing aqueous 70% ethanol, 20 g of the wheat bran was carefully added with vigorous stirring (solids/liquids = 1/5). With continued stirring the mixture was removed from the heat and allowed to extract for an additional 20 min., then poured into a volumetrically graduated column fitted with a polypropylene fritted disk of medium porosity. The column was then refrigerated at 4°C in the dark and allowed to settle by gravity overnight. After re-equilibration to room temperature, the packed column of bed volume V_{b} was drained of supernatant and washed with a further 3xV_{b} of fresh 70% ethanol. The extracted bed material was then removed from the column, resuspended in 100 mL boiling 70% ethanol, and the above extraction procedure repeated twice. The combined extracts and washings were combined, concentrated *in vacuo* by rotary evaporation at 40°C to a yellow-brown syrup, (4.22 g; yield: 21.1 %) and stored at -20°C until further processed.

To isolate the non-polar constituents of interest, a steryl ferulate- and 5-n-alk(en)ylresorcinol-enriched fraction was prepared by Hydrophobic Interaction Chromatography using Octyl-Sepharose CL-4B™ as follows: To the yellow-brown syrup prepared above, 40 mL Octyl Sepharose CL-4B™ pre-equilibrated in 95% ethanol was added and the mixture stirred to dissolve the extract and slurry the gel (absorption ratio: ∼10 mL gel/g extract or ∼2 mL gel/g wheat bran extracted). The slurry was then concentrated *in vacuo* at 40°C by rotary evaporation to remove as much of the solvent as possible, slurried again in aqueous 50% ethanol and carefully added to a 200 mL volumetrically graduated column containing a 60 mL Octyl Sepharose CL-4B™ gel bed, pre-equilibrated and gravity-packed in aqueous 25% ethanol. The gel bed was pre-equilibrated and packed in 25% rather than 50% ethanol to increase the density of the chromatography gel bed relative to the slurried extract-gel mixture to ensure proper gravity packing. After settling, the combined absorbent gel layer and prepacked gel bed volume (i.e. V_{b}) was ∼ 100 mL. The supernatant liquid above the newly constituted 100 mL gel bed was drawn off, the column washed with a further 2xV_{b} of fresh aqueous 50% ethanol and the combined eluate and washings concentrated *in vacuo* at 40° C by rotary evaporation to give a flavonoid-enriched hydrophilic fraction. The alk(en)ylresorcinol- and steryl ferulate-enriched hydrophobic fraction absorbed on the gel was then recovered with 2xV_{b} of 100% isopropanol.

Since the flavonoids present in the flavonoid-enriched hydrophilic fraction are uncharged at or below pH 6, they will not be retained on an anionic exchange matrix under these conditions. However, since the phenolic hydroxyl group(s) of flavonoids are negatively charged at or above pH 8, they will be retained at or above this pH. Accordingly, the hydrophilic fraction was further purified by the following double ion exchange chromatography on QAE Sephadex A-25™ anion exchange columns: First, treatment to remove non-flavonoid components such as anionic prolamines, peptides, and amino acids, organic acids and inorganic anions, all of which carry a net anionic charge at or below pH 6 was performed. A QAE Sephadex A-25™ anion exchange column was prepared essentially as described in Example 5 except it was converted from the hydroxyl form to the acetate form by passing an excess of 5 % glacial acetic acid in aqueous 50% ethanol (ethanol:water:glacial acetic acid 50:45:5 v:v:v) through the column. The column was then equilibrated and washed with aqueous 50% ethanol until the pH of the washings was ∼6. The flavonoid-enriched hydrophilic fraction prepared above was dissolved in ∼10 mL 50% ethanol and absorbed onto the column. The column was then washed with 2xV_{b} of fresh aqueous 50% ethanol and the washing containing the flavonoids and other neutral/cationic components of this sub-fraction evaporated to a thick, yellow glass. This sub-fraction was then redissolved in ∼10 mL aqueous 50% ethanol. A QAE Sephadex A-25™ anion exchange column in the hydroxyl form was prepared essentially as described in Example 5. The sub-fraction was then added to the column and the column washed with a further 2xV_{b} of fresh aqueous 50% ethanol to give a neutral/cationic fraction devoid of flavonoids (HPLC, TLC). The flavonoids were then recovered from the column using the solvent ethanol:water:acetic acid 50:49:1 (v:v:v), concentrated to dryness *in vacuo* at 40°C and stored at -20°C until further analyzed.

Using the same principles of selective ionization of phenolic OH groups at different pH values as described above for the flavonoids and the same protocols as described in Example 5, except that the trifluoracetate-form instead of the acetate-form, was used. The alk(en)ylresorcinol- and steryl ferulate-enriched lipophilic fraction was also further purified by double ion exchange chromatography on QAE Sephadex A-25™ in the trifluoroacetate and the hydroxyl forms. The recovered alk(en)ylresorcinol- and steryl ferulate- sub-fraction was concentrated *in vacuo* at 40°C by rotary evaporation and stored at -20°C until further analyzed. The entire process for isolation, recovery and purification of the flavonoids, al(en)ylresorcinois and steryl ferulates from the wheat bran is summarized in Figure 7.

The structural identification, qualitative and quantitative analyses of the flavonoids, alk(en)ylresorcinols and steryl ferulates were carried out using the following methods:

### High Performance Liquid Chromatography (HPLC):

Semi-preparative HPLC was carried out on a Beckman 110A pump fitted with a Rheodyne 7125 injector and a 200µL sample loop. The HPLC was run in an isocratic mode with 100% methanol at 2.0 mL/min flow rate through a CSC ODS-2 column (10µm, 9.4x250 mm). Injection volume was 50µL (i.e. 8.4 mg) into the 200 µL sample loop. The eluent was monitored at 325 nm for the steryl ferulates and 280 nm for the 5-n-alk(en)ylresorcinols with a Hewlett-Packard 8452 diode array spectrophotometer.

Analytical runs were performed on a Thermo Separation Products P4000 quaternary pump fitted with a Rheodyne 7125 injector and 20 µL sample loop. For both the 5-n-alk(en)ylresorcinols and the steryl ferulates, separations were effected on a CSC ODS-2 column (5µm, 4.0x250 mm), using a mobile phase of methanol:acetonitrile:isopropanol:water (45:45:5:5 v:v:v:v) which was ramped to methanol:acetonitrile:isopropanol (45:50:5 v:v:v) from 6 to 10 minutes and held constant for 30 minutes. The flow rate was 1.0 mL/min and the temperature was maintained at 20°C with a Varian 2080 column oven. For the flavonoids, analytical separations were carried out on a Hypersil ODS column (5µm, 4.6x250 mm), using a mobile phase of acetonitrile and aqueous 2% acetic acid at a flow rate of 1.0mL/min and the following gradient (v:v):

| Time | Acetonitrile | 2%Acetic Acid |
|---|---|---|
| 0 | 9 | 91 |
| 15 | 15 | 85 |
| 25 | 15 | 85 |
| 35 | 30 | 70 |
| 45 | 30 | 70 |
| 47 | 80 | 20 |
| 50 | 9 | 91 |

### Thin-Layer Chromatography:

Thin-layer chromatography of steryl ferulates and 5-n-alk(en)ylresorcinols was performed on both silica gel plates (Baker-Flex, 1B2-F), in the normal phase mode, with n-butyl acetate:cyclohexane (20:80 v:v) as solvent, and C₁₈ precoated plates (Whatman, MK C₁₈ F, 200 µm) with 100% methanol as solvent. Steryl ferulates were visualized by examination of the plates under UV (365nm) before and after spraying with a 5 % solution of ethanolamine in isopropanol. The steryl ferulates were visible as dark UV-absorbing spots before spraying, turning sky blue with ethanolamine. The 5-n-alk(en)ylresorcinols were visualised as brick red spots with the *p*-anisaldehyde spray reagent described in Example 1.

Thin-layer chromatography of the flavonoids was carried out on silica gel plates (Baker-Flex, 1B2-F), in the normal phase mode, using the solvent system: CH₂Cl₂:ethyl acetate:methanol:aqueous 5%acetic acid 40:35:20:5 (v:v:v:v), and visualized by examination of the plate under UV light (365nm) before and after spraying with a solution of 5% ethanolamine in isopropanol. Specific flavonoids gave yellow-green fluorescent colors characteristic of their structure and substitution patterns.

### Spectral Analyses:

UV data was provided by a Waters 991 Photodiode Array Detector. NCI mass spectra were obtained using flow injection on a Hewlett Packard 1090 HPLC coupled to a 5988 HP mass spectrometer via a thermospray interface. EI mass spectra were acquired by direct inlet solid probe analysis on a Finnigan 4500 spectrometer with an ionization potential set to 30 eV. NMR spectra were acquired on a Bruker AM 500 spectrometer operating at 500 MHZ and 125.7 MHZ, respectively, for ¹H and ¹³C. Spectra were recorded in CDCl₃ (steryl ferulates and alk(en)ylresorcinols) or DMSO-d₆ (flavonoids) and referenced to CHCl₃ at 7.24 ppm (¹H) and CDCl₃ at 77.0 ppm (¹³C). ¹H assignments were aided by ¹H/¹³C heteronuclear correlation spectra (HETCORR) and carbon resonance assignments by DEPT and HETCORR NMR experiments. Melting points were recorded on an Electrothermal apparatus and are uncorrected.

### Quantitative Analyses:

Quantization of the steryl ferulates was carried out as described in Example 5. The response factor was applied to all types of steryl ferulates and consequently any concentrations are reported in terms of 24-methylenecycloartanol-3β-O-E-ferulate equivalents. Quantisation of the 5-n-alk(en)ylresorcinols was similarly carried out using 5-n-pentadecylresorcinol (Aldrich Chemical Co., Milwaukee, WI.) as standard and preparing the calibration curve based on 280nm absorptivity. Quantisation of flavonoids was recorded as flavone di-C-glycoside equivalents, based on a response calibration curve prepared with a crystalline standard of Apigenin-6C-α-L-arabinofuranosyl-8C-β-D-pyranosylgalactoside obtained from wheat germ (Collins, F. W. and D'Attilio, R.Z., Cereal Foods World 41: 586, 1996). All standards and samples were stored at 4°C in the dark and were stable for several months (no detectable indication of E/Z isomerization or auto-oxidation).

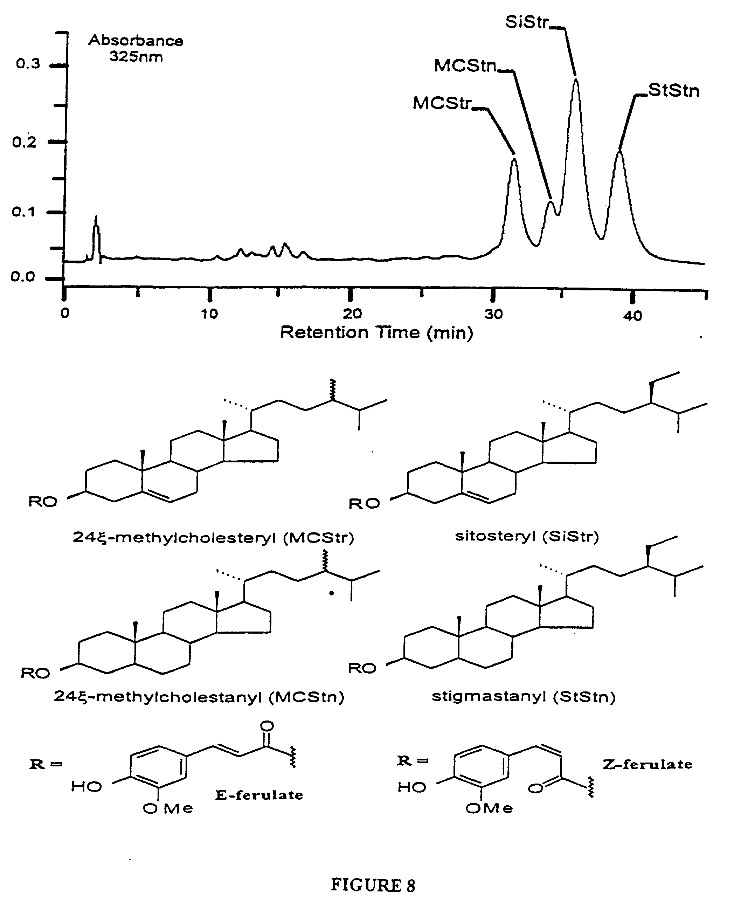

A typical HPLC profile of the mixture of steryl ferulates prepared from the wheat bran is shown in Figure 8. Structural identification of the individual components was made by co-chromatography (HPLC, TLC) and comparison of spectral data (UV, MS, NMR) with authentic standards. Unlike the rice bran extracts (Figure 6), only Type II steryl ferulates were observed in the wheat bran extracts. In all, 4 major steryl ferulates were identified. The major component identified was the E-isomer of sitosteryl ferulate (SiStr), followed by the E-isomer of stigmastanyl ferulate (StStn). Lesser relative levels of both 24ξ-methylcholesteryl (MCStr) and 24ξ-methylcholestanyl (MCStn) ferulates in the E-forms were also detected. When exposed to light and moisture, all these ferulates undergo reversible isomerization (to various degrees depending on the individual compound involved and the conditions of light, pH, aqueous hydration etc.) and a mixture of both E- and Z-isomers can be encountered. However, no Z-isomers of the individual ferulates were detected under the preparation and storage conditions used in this example.

The results of the quantitative analyses of individual steryl ferulates are summarized in Table 3. When expressed as 24-methylenecycloartenol, the total quantity of steryl ferulates was 58.4 mg/100gm of wheat bran, or 584 ppm (dry basis).

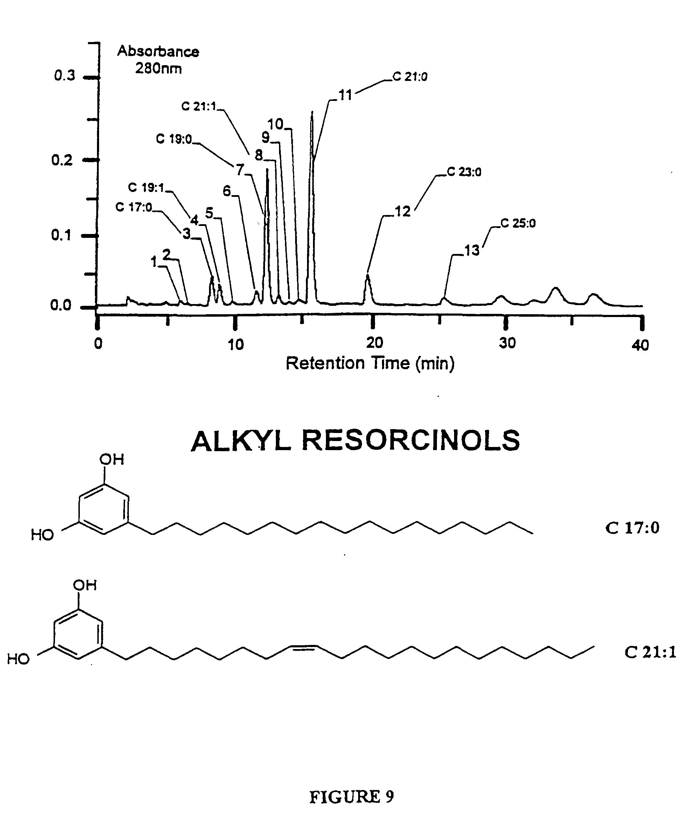

The 5-n-alk(en)ylresorcinol HPLC showed the presence of at least 13 peaks 12 of which were identified as members of this group of phenolic antioxidants. Individual components of the group were identified as described by Collins, F.W. and Mullin, W.J., J. Food Comp. Anal. 4: 270-275, 1991. An HPLC profile of the alk(en)ylresorcinol mixture from the wheat bran is shown in Figure 9, along with the identity of major components and structural examples. The structural examples indicate the position of attachment of the alkyl or alkenyl function represented by the abbreviation used in the HPLC profile (e.g. C 17:0, C 21:1 etc.). The results of the quantitative analysis are summarized in Table 4. The profile was dominated by 3 major components (peak #7, #11 and #12, Figure 9) in the homologous series containing n-alkyl side chains of 19, 21 and 23 carbons. Lesser amounts of the corresponding mono- and di-unsaturated analogues were also present. As shown in Table 3, the total amount of 5-n-alk(en)ylresorcinols in the wheat bran sample, calculated by summation and expressed as the 5-n-pentadecylresorcinol equivalents, was about 600 mg/gm of bran extracted or 6,000 ppm (dry basis).

Since the steryl ferulates and 5-n-alk(en)ylresorcinols were not group separated from each other in this example, the overall purity of each group could not be evaluated directly by comparing the spectrophotometric quantisation with the gravimetric quantisation for each fraction. Nevertheless, based on summation of the UV data of the combined fractions with the total weight of the lipophilic sub-fraction used in the spectrophotometric quantisations (Tables 3 and 4), overall purity was estimated at over 95% with respect to these 2 groups of compounds.

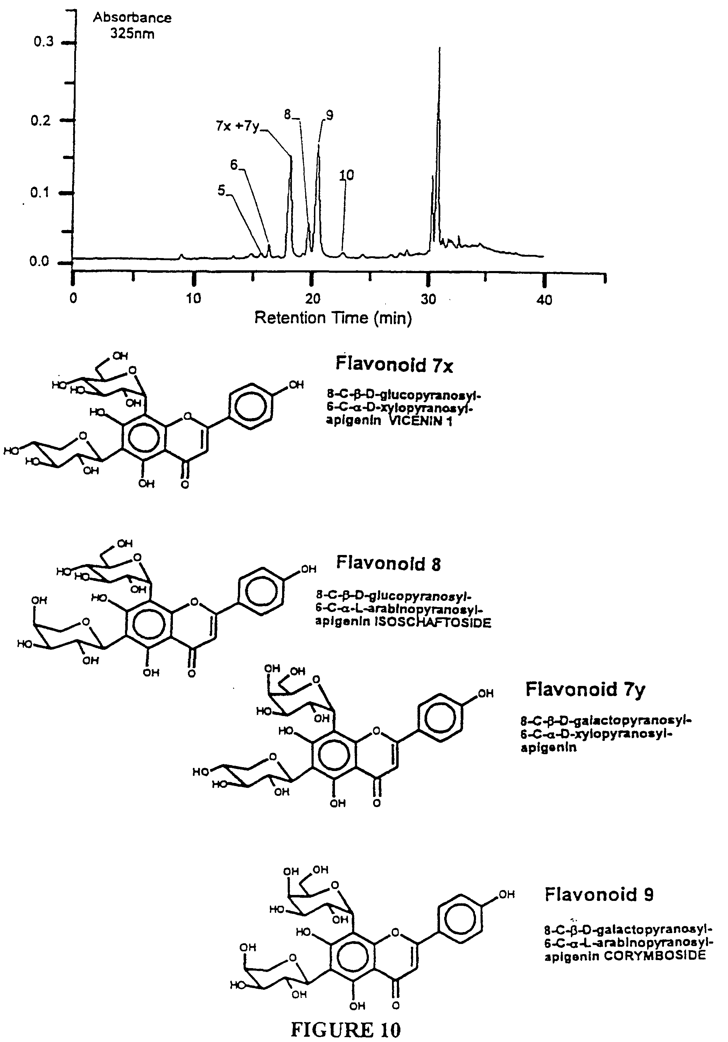

The results of the flavonoid HPLC analysis are shown in Figure 10, along with the structures of the major components of the mixture. The 4 major glycosides identified (HPLC, TLC, NMR, MS) included: 7x (Vicenin I), 7y (8-C-β-D-galactopyranosyl-6-C-β-xylopyranosyl-apigenin), 8, {8-β-D-glucopyranosyl-α-L-arabinosyl-apigenin (Isoshaftoside)}, and 9, {8-β-D-galactopyranosyl-α-L-arabinosyl-apigenin (Corymboside)}. Although 7x and 7y were not separated in the profile shown, they were detected in other HPLC systems. A number of other flavonoids were detected and monitored but their structures have not been confirmed. These included flavonoids 5, 6 and 10. Total flavonoids expressed as mg equivalents of flavonoid 9 was 2.0 mg/100gm or 20 ppm (dry basis).

**TABLE 3**

| **Composition of Type I Steryl Ferulates from Wheat Bran** | | |
|---|---|---|
| Compound | RRₜ* | Concentration mg/100g** |
| 24ξ-methylcholesteryl-*Z*-ferulate | 1.088 | nd |
| 24ξ-methylcholesteryl-*E*-ferulate | 1.110 | 12.0 |
| 24ξ-methylcholestanyl-*Z*-ferulate | 1.186 | nd |
| 24ξ-methylcholestanyl-*E*-ferulate | 1.210 | 24.7 |
| Sitosteryl-*Z*-ferulate | 1.234 | nd |
| Sitosteryl-*E*-ferulate | 1.268 | 5.4 |
| Stigmastanyl-*Z*-ferulate | 1.341 | nd |
| Stigmastanyl-*E*-ferulate | 1.388 | 16.3 |
| | | |
| Total Type I steryl ferulates | | 58.4 |

| | | |
|---|---|---|
| * Relative Retention Times (RRₜ) relative to 24-methylenecycloartanol-*E*-ferulate (absolute retention time =28.10 min) | | |
| ** Concentration reported in 24-methylenecycloartenol-*E*-ferulate equivalents; nd not detected | | |

**TABLE 4**

| **Composition of 5-n-alk(en)ylresorcinols from Wheat Bran** | | | |
|---|---|---|---|
| Peak No. | 5-n-Substituent | RRₜ* | Concentration mg/100 g** |
| 1 | C 15:0 | 1.000 | 5.9 |
| 2 | C 19:2 | 1.113 | 1.7 |
| 3 | C 17:0 | 1.270 | nd |
| 4 | C 19:1 | 1.327 | 28.4 |
| 5 | C 21:2 | 1.434 | 4.0 |
| 6 | C 21:1 | 1.523 | 20.1 |
| 7 | C 19:0 | 1.531 | 178.8 |
| 8 | C 23:1 | 1.866 | 9.8 |
| 9 | | 1.988 | nd |
| 10 | C 25:1 | 2.104 | nd |
| 11 | C 21:0 | 2.187 | 276.3 |
| 12 | C 23:0 | 2.790 | 54.5 |
| 13 | C 25:0 | 3.658 | 19.3 |
| Total 5-n-alk(en)ylresorcinols | | | 598.4 |

| | | | |
|---|---|---|---|
| * Relative Retention Times (RRₜ) relative to 5-n-pentadecylresorcinol (absolute retention time = 6.63min) | | | |
| ** Concentration reported in 5-n-pentadecylresorcinol equivalents nd not determined | | | |

All scientific publications and patent documents are incorporated herein by reference.

The present invention has been described with regard to preferred embodiments. However, it will be understood to persons skilled in the art that a number of variations and modifications can be made without departing from the scope of the invention as described in the following claims.

## Claims

1. A method of isolating a non-polar extractive comprising:
contacting said non-polar extractive in an aqueous organic solvent with an aliphatic-substituted polysaccharide gel matrix;
washing said gel matrix with a first aqueous organic solvent solution;
optionally recovering a first extractive from a first effluent stream;
washing said gel matrix with a second aqueous organic solvent solution, wherein the proportion of the organic solvent in said second solution is increased over the proportion of the organic solvent in said first aqueous organic solvent; and
recovering said second extractive from a second effluent stream,
wherein the gel matrix consists of a neutral polysaccharide gel matrix of the polyanhydrogalactan class, a neutral polysaccharide gel matrix of the polydextran class or a neutral polysaccharide gel matrix of the hydroxypropyl polydextran class.

2. The method of claim 1, further comprising regenerating the gel matrix for re-use.

3. The method of claim 1 or claim 2 wherein said non-polar extractive is a non-polar plant extractive.

4. The method according to claim 3, wherein the non-polar plant extractives are selected from the group consisting of steroids and triterpenoids, flavonoids, phenolic conjugates, polar lipids, and prolamines.

5. The method according to claim 4 , wherein the steroids and triterpenoids are selected from the group consisting of saponins, cardiac glycosides and steryl conjugates.

6. The method according to claim 4, wherein the flavonoids are selected from the group consisting of flavones, flavonols, isoflavones and all of their glycosides.

7. The method according to claim 4, wherein the conjugate portion of phenolic conjugates is selected from the group consisting of aliphatic alcohols, esters and amides.

8. The method according to claim 4, wherein the polar lipids are selected from the group consisting of mono- and di-glycerides and their derivatives and alk(en)yl resorcinols.

9. The method according to claim 4, wherein the prolamines are selected from the group consisting of zein, avenin, hordein and gliadin.

10. The method according to claim 1, wherein the non-polar extractives are from either synthetic or natural source.

11. The method according to claim 10, wherein the natural source of the non-polar extractives is selected from the group consisting of plant material, algae, fungi and unicellular organisms.

12. The method according to claim 11, wherein the plant material is selected from the group consisting of agricultural, viniculture, horticulture, aquaculture and plants native to lands and oceans.

13. The method according to claim 12, wherein the agriculture plant material is selected from the group consisting of wheat, oats, rye, corn, rice, quinoa, amaranth, buckwheat, triticale or barley; or oilseeds, such as soybean, canola, flaxseed, sunflower, safflower or mustard; or pulse crops, for example, peas, lentils or beans; or forage crops, such as fescue, timothy, clover, alfalfa or wheatgrass; or herbs, such as parsley, rosemary, sage or mint.

14. The method according to claim 1, wherein the organic solvent solution is a solution containing a lower alcohol, a ketone or a combination thereof.

15. The method according to claim 14, wherein the lower alcohol is selected from the group consisting of methanol, ethanol, propanol and isopropanol.

16. The method according to claim 14, wherein the ketone is acetone.

17. The method according to claim 1, wherein the gel matrix contains covalently linked alkyl substitution of from 4 carbon (ie butyl) to 8 carbon (ie octyl) functions at a 4% or more substitution rate, stable in neutral and mildly acidic or alkaline solutions of said aqueous organic solvent solution, with a molecular size exclusion cut-off range equal to or greater than 10,000 Daltons.

18. The method according to claim 17, wherein the substitution rate is from 4% to 8%.

19. The method according to claim 18, wherein the substitution rate is from 4% to 6%.

20. The method according to claim 18, wherein the gel matrix is Octyl Sepharose CL-4B™ gel.

## Patentansprüche

1. Verfahren zur Isolierung eines unpolaren Extraktivstoffs, das umfaßt:
Inkontaktbringen des unpolaren Extraktivstoffs in einem wäßrigen organischen Lösemittel mit einer aliphatisch-substituierten Polysaccharidgel-Matrix;
Waschen der Gelmatrix mit einer ersten Lösung eines wäßrigen organischen Lösemittels, wobei man ggf. einen ersten Extraktivstoff aus einem ersten Abflußstrom gewinnt;
Waschen der Gelmatrix mit einer zweiten Lösung eines wäßrigen organischen Lösemittels, wobei der Anteil des organischen Lösemittels in der zweiten Lösung gegenüber dem Anteil des organischen Lösemittels in dem ersten wäßrigen organischen Lösemittel erhöht ist; und
Gewinnen des zweiten Extraktivstoffs aus einem zweiten Abflußstrom,
wobei die Gelmatrix aus einer neutralen Polysaccharidgel-Matrix der Polyanhydrogalactan-Klasse, einer neutralen Polysaccharidgel-Matrix der Polydextran-Klasse oder einer neutralen Polysaccharidgel-Matrix der Hydroxypropyl-Polydextran-Klasse besteht.

2. Verfahren nach Anspruch 1, das außerdem die Regenerierung der Gelmatrix zur Wiederverwendung umfaßt.

3. Verfahren nach Anspruch 1 oder Anspruch 2, wobei der unpolare Extraktivstoff ein unpolarer pflanzlicher Extraktivstoff ist.

4. Verfahren nach Anspruch 3, wobei die unpolaren pflanzlichen Extraktivstoffe aus der Gruppe ausgewählt sind, die besteht aus Steroiden und Triterpenoiden, Flavonoiden, phenolischen Konjugaten, polaren Lipiden und Prolaminen.

5. Verfahren nach Anspruch 4, wobei die Steroide und Triterpenoide aus der Gruppe ausgewählt sind, die besteht aus Saponinen, Herzglycosiden und Sterylkonjugaten.

6. Verfahren nach Anspruch 4, wobei die Flavonoide aus der Gruppe ausgewählt sind, die besteht auf Flavonen, Flavonolen, Isoflavonolen, und allen ihren Glycosiden.

7. Verfahren nach Anspruch 4, wobei der Konjugatteil der phenolischen Konjugate aus der Gruppe ausgewählt ist, die besteht aus aliphatischen Alkoholen, Estern und Amiden.

8. Verfahren nach Anspruch 4, wobei die polaren Lipide aus der Gruppe ausgewählt sind, die besteht aus Mono- und DiGlyceriden und ihren Derivaten und Alk(en)ylresorcinolen.

9. Verfahren nach Anspruch 4, wobei die Prolamine aus der Gruppe ausgewählt sind, die besteht aus Zein, Avenin, Hordein und Gliadin.

10. Verfahren nach Anspruch 1, wobei die unpolaren Extraktivstoffe entweder synthetischer oder natürlicher Herkunft sind.

11. Verfahren nach Anspruch 10, wobei die natürliche Quelle für die unpolaren Extraktivstoffe aus der Gruppe ausgewählt ist, die besteht aus pflanzlichem Material, Algen, Pilzen und einzelligen Organismen.

12. Verfahren nach Anspruch 11, wobei das Pflanzenmaterial aus der Gruppe ausgewählt ist, die besteht aus landwirtschaftlichen, weinwirtschaftlichen, gartenwirtschaftlichen, wasserwirtschaftlichen Pflanzen und Pflanzen, die an Land und in den Ozeanen heimisch sind.

13. Verfahren nach Anspruch 12, wobei das landwirtschaftliche pflanzliche Material aus der Gruppe ausgewählt ist, die besteht aus Weizen, Hafer, Roggen, Mais, Reis, Quinoa, Amaranth, Buchweizen, Tritikale oder Gerste; oder Ölsaaten wie Sojabohnen, Canola, Leinsamen, Sonnenblumen, Safflor oder Senf; oder Hülsenfrüchten, beispielsweise Erbsen, Linsen oder Bohnen; oder Futterpflanzen wie Schwingel, Timothygras, Klee, Luzerne oder Quecke; oder Kräutern wie Petersilie, Rosmarin, Salbei oder Minze.

14. Verfahren nach Anspruch 1, wobei die Lösung des organischen Lösemittels eine Lösung ist, die einen niedrigen Alkohol, ein Keton oder eine Kombination davon enthält.

15. Verfahren nach Anspruch 14, wobei der niedrige Alkohol aus der Gruppe ausgewählt ist, die besteht aus Methanol, Ethanol, Propanol und Isopropanol.

16. Verfahren nach Anspruch 14, wobei das Keton Aceton ist.

17. Verfahren nach Anspruch 1, wobei die Gelmatrix eine kovalent gebundene Alkylsubstitution mit von 4 Kohlenstoff-(d.h. Butyl) bis 8 Kohlenstoff-(d.h. Octyl)-Funktionen bei einem Substitutionsgrad von 4 % oder mehr aufweist, die in neutralen oder schwach sauren oder alkalischen Lösungen der wäßrigen Lösung des organischen Lösemittels stabil sind, wobei sie einen Molekülgrößen-Ausschlußbereich von gleich oder größer als 10.000 Dalton aufweist.

18. Verfahren nach Anspruch 17, wobei der Substitutionsgrad von 4 % bis 8 % beträgt.

19. Verfahren nach Anspruch 18, wobei der Substitutionsgrad von 4 % bis 6 % beträgt.

20. Verfahren nach Anspruch 18, wobei die Gelmatrix ein Octylsepharose CL-4B™-Gel ist.

## Revendications

1. Méthode d'isolement d'un extrait non polaire comprenant :
le fait de mettre en contact ledit extrait non polaire dans un solvant aqueux organique avec une matrice de gel de polysaccharide substitué par des groupes aliphatiques ;
le fait de laver ladite matrice de gel avec une première solution aqueuse de solvant organique ; le fait de récupérer éventuellement un premier extrait à partir d'un premier flux d'effluent ; le fait de laver ladite matrice de gel avec une seconde solution aqueuse de solvant organique, dans laquelle la proportion de solvant organique dans ladite seconde solution est accrue par rapport à la proportion de solvant organique dans ledit premier solvant organique aqueux ; et
le fait de récupérer ledit second extrait à partir d'un second flux d'effluent,
dans laquelle la matrice de gel consiste en une matrice de gel de polysaccharide neutre de la classe du polyanhydrogalactane, une matrice de gel de polysaccharide neutre de la classe du polydextran ou une matrice de gel de polysaccharide neutre de la classe de l'hydroxypopylprolydextran.

2. Méthode de la revendication 1, comprenant en outre le fait de régénérer la matrice de gel pour la réutiliser.

3. Méthode de la revendication 1 ou de la revendication 2 dans laquelle ledit extrait non polaire est un extrait non polaire de plante.

4. Méthode selon la revendication 3, dans laquelle les extraits non polaires de plante sont choisis dans le groupe constitué de stéroïdes et de triterpénoïdes, de flavonoïdes, de conjugués phénoliques, de lipides polaires et de prolamines.

5. Méthode selon la revendication 4, dans laquelle les stéroïdes et les triterpénoïdes sont choisis dans le groupe constitué de saponines, de glycosides cardiaques et de conjugués de stéryle.

6. Méthode selon la revendication 4 , dans laquelle les flavonoïdes sont choisis dans le groupe constitué de flavones, de flavonols, d'isoflavones et de tous leurs glycosides.

7. Méthode selon la revendication 4, dans laquelle la portion conjuguée des conjugués phénoliques est choisie dans le groupe constitué d'alcools, esters et amides aliphatiques.

8. Méthode selon la revendication 4, dans laquelle les lipides polaires sont choisis dans le groupe constitué de mono- et di-glycérides et de leurs dérivés et d'alk(én)yl-résorcinols.

9. Méthode selon la revendication 4, dans laquelle les prolamines sont choisies dans le groupe constitué de zéine, avénine, hordéine et gliadine.

10. Méthode selon la revendication 1, dans laquelle les extraits non polaires sont de source synthétique ou naturelle.

11. Méthode selon la revendication 10, dans laquelle la source naturelle des extraits non polaires est choisie dans le groupe constitué de matériel végétal, algues, champignons et organismes unicellulaires.

12. Méthode selon la revendication 11, dans laquelle le matériel végétal est choisi dans le groupe provenant de l'agriculture, viticulture, horticulture, aquaculture et de plantes originaires des terres et des océans.

13. Méthode selon la revendication 12, dans laquelle le matériel végétal agricole est choisi dans le groupe constitué de blé, avoine, seigle, maïs, riz, quinoa, amarante, sarrasin, triticale ou orge ; ou d'oléagineux, tels que soja, canola, lin, tournesol, carthame ou moutarde ; ou de légumineuses, par exemple, pois, lentilles ou haricots ; ou de plantes fourragères, telles que fétuque, fléole, trèfle, luzerne ou chiendent ; ou de plantes condimentaires, telles que persil, romarin, sauge ou menthe.

14. Méthode selon la revendication 1, dans laquelle la solution de solvant organique est une solution contenant un alcool inférieur, une cétone ou une combinaison de ceux-ci.

15. Méthode selon la revendication 14, dans laquelle l'alcool inférieur est choisi dans le groupe constitué du méthanol, éthanol, propanol et isopropanol.

16. Méthode selon la revendication 14, dans laquelle la cétone est l'acétone

17. Méthode selon la revendication 1, dans laquelle la matrice de gel contient une substitution alkyle liée par une liaison covalente de fonctions allant de 4 carbones (c'est-à-dire butyle) à 8 carbones (c'est-à-dire octyle) avec un taux de substitution de 4 % ou plus, stable dans des solutions neutres et faiblement acides ou alcalines de ladite solution aqueuse de solvant organique, avec une taille moléculaire de coupure dans une gamme égale ou supérieure d'exclusion 10 000 daltons.

18. Méthode selon la revendication 17, dans laquelle le taux de substitution est de 4 % à 8 %.

19. Méthode selon la revendication 18, dans laquelle le taux de substitution est de 4 % à 6 %.

20. Méthode selon la revendication 18, dans laquelle la matrice de gel est le gel Octyl Sepharose CL-4B™.
